# EUROPÄISCHE PATENTSCHRIFT

(11) **EP 1 286 991 B1**
(45) Veröffentlichungstag und Bekanntmachung des Hinweises auf die Patenterteilung: **06.06.2007**
(21) Anmeldenummer: 00989895.8
(22) Anmeldetag: 27.11.2000
(51) Int. Cl.: C07D 413/10, A01N 43/72, C07D 261/20

(54) **CYCLOPROPYL-ANELLIERTE 3-(4,5-DIHYDROISOXAZOL-3-YL)-SUBSTITUIERTE BENZOYLPYRAZOLE ALS HERBIZIDE**
CYCLOPROPYL-ANELLATED 3-(4,5-DIHYDROISOXAZOL-3-YL)-SUBSTITUED BENZOYLPYRAZOLES
BENZOYLPYRAZOLES SUBSTITUES EN 3-(4,5-DIHYDROISOXAZOL-3-YLE) ET A ANELLATION CYCLOPROPYLE

(30) Priorität: 02.12.1999 DE 19958031
(43) Veröffentlichungstag der Anmeldung: 05.03.2003
(73) Patentinhaber: BASF Aktiengesellschaft, 67056 Ludwigshafen (DE)
(72) Erfinder: KUDIS, Steffen, 68167 Mannheim (DE); BAUMANN, Ernst, 67373 Dudenhofen (DE); VON DEYN, Wolfgang, 67435 Neustadt (DE); LANGEMANN, Klaus, 34270 Schauenburg (DE); MAYER, Guido, 67161 Gönnheim (DE); MISSLITZ, Ulf, 67433 Neustadt (DE); NEIDLEIN, Ulf, 68165 Mannheim (DE); WITSCHEL, Matthias, 67098 Bad Dürkheim (DE); WESTPHALEN, Karl-Otto, 67346 Speyer (DE); WALTER, Helmut, 67283 Obrigheim (DE)
(86) Internationale Anmeldenummer: PCT/EP2000/011823
(87) Internationale Veröffentlichungsnummer: WO 2001/040221

(56) Entgegenhaltungen:
- WO-A-96/26206
- WO-A-98/31681
- WO-A-98/31682
- WO-A-99/21852
- WO-A-99/23094
- WO-A-99/26930
- WO-A-99/63823

## Beschreibung

Die vorliegende Erfindung betrifft bestimmte Cyclopropyl-anellierte 3-(4,5-Dihydroisoxazol-3-yl)-substituierte Benzoylpyrazole sowie Zwischenprodukte und Verfahren zu ihrer Herstellung, Mittel, welche diese enthalten, sowie die Verwendung dieser Derivate oder diese enthaltende Mittel zur Schadpflanzenbekämpfung.

Aus der Literatur, beispielsweise aus WO 96/26206, WO 98/31682 und WO 98/31681, sind Pyrazol-4-yl-benzoylderivate bekannt.

Die älteren Anmeldungen WO 00/34273, WO 00/34272, DE 19936520.2 und DE 19936518.0 beschreiben inter alia (4,5-Dihydroisoxazol-3-yl)-substituierte Benzoylpyrazole und deren herbiziden Eigenschaften. Derivate mit anellierten Cycloalkanringen sind nicht beschrieben.

Die herbiziden Eigenschaften der bisher bekannten Verbindungen sowie die Verträglichkeiten gegenüber Kulturpflanzen können jedoch nur bedingt befriedigen. Es lag daher dieser Erfindung die Aufgabe zugrunde, neue, insbesondere herbizid wirksame, Verbindungen mit verbesserten Eigenschaften zu finden.

Demgemäß wurden Cyclopropyl-anellierte 3-(4,5-Dihydroisoxazol-3-yl)-substituierte Benzoylpyrazole der Formel I 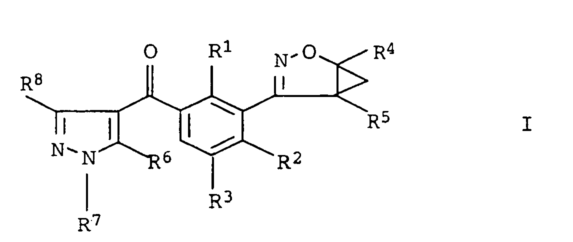 worin
- R¹: für C₁-C₆-Alkyl, C₁-C₆-Halogenalkyl, C₁-C₆-Alkoxy, C₁-C₆-Halogenalkoxy, Halogen oder Nitro;
- R²: für C₁-C₆-Alkyl, C₁-C₆-Halogenalkyl, C₁-C₆-Alkoxy, C₁-C₆-Halo-genalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, Halogen, Cyano oder Nitro;
- R³: für Wasserstoff, C₁-C₆-Alkyl oder Halogen;
- R⁴: für Wasserstoff, C₁-C₄-Alkyl oder C₁-C₄-Halogenalkyl steht;
- R⁵: die für R⁴ angegebenen Bedeutungen einnehmen kann; oder
- R⁴,: R⁵ gemeinsam für eine C₁-C₄-Alkandiylgruppe stehen, die partiell oder vollständig halogeniert sein kann und/oder eine bis drei C₁-C₄-Alkylgruppen tragen kann;
- R⁶: für Hydroxy, C₁-C₆-Alkoxy, C₃-C₆-Alkenyloxy, C₁-C₆-Alkylsulfonyloxy, C₁-C₆-Alkylcarbonyloxy, Phenyl-C₁-C₄-alkoxy, Phenylcarbonyl-C₁-C₄-alkoxy, Phenylsulfonyloxy, Phenylcarbonyloxy, wobei der Phenylrest der vier letztgenannten Substituenten partiell oder vollständig halogeniert sein kann und/oder eine bis drei der folgenden Gruppen tragen kann: Nitro, Cyano, C₁-C₄-Alkyl, C₁-C₄-Halogenalkyl, C₁-C₄-Alkoxy oder C₁-C₄-Halo-genalkoxy;
- R⁷: für Wasserstoff, C₁-C₆-Alkyl oder Cyclopropyl;
- R⁸: für Wasserstoff, C₁-C₆-Alkyl oder C₁-C₆-Halogenalkyl steht;
sowie deren landwirtschaftlich brauchbaren Salze gefunden.

Ferner wurden herbizide Mittel gefunden, die die Verbindungen I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I gefunden.

Die Verbindungen der Formel I können je nach Substitutionsmuster ein oder mehrere Chiralitätszentren enthalten und liegen dann als Enantiomeren- oder Diastereomerengemische vor. Gegenstand der Erfindung sind sowohl die reinen Enantiomeren oder Diastereomeren als auch deren Gemische.

Die Verbindungen der Formel I können auch in Form ihrer landwirtschaftlich brauchbaren Salze vorliegen, wobei es auf die Art des Salzes in der Regel nicht ankommt. Im allgemeinen kommen die Salze derjenigen Kationen oder die Säureadditionssalze derjenigen Säuren in Betracht, deren Kationen, beziehungsweise Anionen, die herbizide Wirkung der Verbindungen I nicht negativ beeinträchtigen.

Es kommen als Kationen, insbesondere Ionen der Alkalimetalle, vorzugsweise Lithium, Natrium und Kalium, der Erdalkalimetalle, vorzugsweise Calcium und Magnesium, und der Übergangsmetalle, vorzugsweise Mangan, Kupfer, Zink und Eisen, sowie Ammonium, wobei hier gewünschtenfalls ein bis vier Wasserstoffatome durch C₁-C₄-Alkyl, Hydroxy-C₁-C₄-alkyl, C₁-C₄-Alkoxy-C₁-C₄-alkyl, Hydroxy-C₁-C₄-alkoxy-C₁-C₄-alkyl, Phenyl oder Benzyl ersetzt sein können, vorzugsweise Ammonium, Dimethylammonium, Diisopropylammonium, Tetramethylammonium, Tetrabutylammonium, 2-(2-Hydroxyeth-1-oxy)eth-1-ylammonium, Di(2-hydroxyeth-1-yl)ammonium, Trimethylbenzylammonium, des weiteren Phosphoniumionen, Sulfoniumionen, vorzugsweise Tri(C₁-C₄-alkyl)sulfonium und Sulfoxoniumionen, vorzugsweise Tri(C₁-C₄-alkyl)sulfoxonium, in Betracht.

Anionen von brauchbaren Säureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von C₁-C₄-Alkansäuren, vorzugsweise Formiat, Acetat, Propionat und Butyrat.

Die für die Substituenten R¹-R⁸ oder als Reste an Phenylringen genannten organischen Molekülteile stellen Sammelbegriffe für individuelle Aufzählungen der einzelnen Gruppenmitglieder dar. Sämtliche Kohlenwasserstoffketten, also alle Alkyl-, Alkylcarbonyl-, Halogenalkyl-, Alkoxy-, Halogenalkoxy-, Alkylcarbonyloxy-, Alkylsulfonyloxy-, Alkylthio-, Halogenalkylthio-, Alkylsulfinyl-, Halogenalkylsulfinyl-, Alkylsulfonyl-, Halogenalkylsulfonyl-, Alkenyl-, Alkenyloxy, Phenylalkyl-, Phenylcarbonylalkyl-, Phenylalkoxy- und Phenylcarbonylalkoxy-Teile können geradkettig oder verzweigt sein. Sofern nicht anders angegeben, tragen halogenierte Substituenten vorzugsweise ein bis fünf gleiche oder verschiedene Halogenatome. Die Bedeutung Halogen steht jeweils für Fluor, Chlor, Brom oder Iod.

Ferner bedeuten beispielsweise:
- C₁-C₄-Alkyl, sowie die Alkylteile von C₁-C₄-Alkylcarbonyl, C₁-C₄-Alkylcarbonyloxy, Phenyl-C₁-C₄-alkyl und Phenylcarbonyl-C₁-C₄-alkyl: z. B. Methyl, Ethyl, Propyl, 1-Methylethyl, Butyl, 1-Methylpropyl, 2-Methylpropyl und 1,1-Dimethylethyl;
- C₁-C₆-Alkyl, sowie die Alkylteile von C₁-C₆-Alkylcarbonyl und C₁-C₆-Alkylcarbonyloxy: C₁-C₄-Alkyl, wie voranstehend genannt, sowie z. B. Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl und 1-Ethyl-3-methylpropyl;
- C₁-C₄-Halogenalkyl: einen C₁-C₄-Alkylrest, wie vorstehend genannt, der partiell oder vollständig durch Fluor, Chlor, Brom und/oder Iod substituiert ist, also z. B. Chlormethyl, Dichlormethyl, Trichlormethyl, Fluormethyl, Difluormethyl, Trifluormethyl, Brommethyl, Iodmethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl und Nonafluorbutyl;
- C₁-C₆-Halogenalkyl: C₁-C₄-Halogenalkyl, wie voranstehend genannt, sowie z. B. 5-Fluorpentyl, 5-Chlorpentyl, 5-Brompentyl, 5-Iodpentyl, Undecafluorpentyl, 6-Fluorhexyl, 6-Chlorhexyl, 6-Bromhexyl, 6-Iodhexyl oder Dodecafluorhexyl;
- C₁-C₂-Alkoxy als Alkoxyteile von Phenyl-C₁-C₂-alkoxy und Phenylcarbonyl-C₁-C₂-alkoxy: Methoxy und Ethoxy;
- C₁-C₄-Alkoxy, sowie die Alkoxyreste von Phenyl-C₁-C₄-alkoxy und Phenylcarbonyl-C₁-C₄-alkoxy: C₁-C₂-Alkoxy, wie voranstehend genannt, sowie z. B. Propoxy, 1-Methylethoxy, Butoxy, 1-Methylpropoxy, 2-Methylpropoxy und 1,1-Dimethylethoxy;
- C₁-C₆-Alkoxy: C₁-C₄-Alkoxy, wie voranstehend genannt, sowie z. B. Pentoxy, 1-Methylbutoxy, 2-Methylbutoxy, 3-Methoxylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy,1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy;
- C₁-C₄-Halogenalkoxy: einen C₁-C₄-Alkoxyrest, wie voranstehend genannt, der partiell oder vollständig durch Fluor, Chlor, Brom und/oder Iod substituiert ist, also z. B. Fluormethoxy, Difluormethoxy, Trifluormethoxy, Chlordifluormethoxy, Bromdifluormethoxy, 2-Fluorethoxy, 2-Chlorethoxy, 2-Bromethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy, 2-Fluorpropoxy, 3-Fluorpropoxy, 2-Chlorpropoxy, 3-Chlorpropoxy, 2-Brompropoxy, 3-Brompropoxy, 2,2-Difluorpropoxy, 2,3-Difluorpropoxy, 2,3-Dichlorpropoxy, 3,3,3-Trifluorpropoxy, 3,3,3-Trichlorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy und Nonafluorbutoxy;
- C₁-C₆-Halogenalkoxy: C₁-C₄-Halogenalkoxy, wie voranstehend genannt, sowie z. B. 5-Fluorpentoxy, 5-Chlorpentoxy, 5-Brompentoxy, 5-Iodpentoxy, Undecafluorpentoxy, 6-Fluorhexoxy, 6-Chlorhexoxy, 6-Bromhexoxy, 6-Iodhexoxy oder Dodecafluorhexoxy;
- C₁-C₄-Alkylthio: z. B. Methylthio, Ethylthio, Propylthio, 1-Methylethylthio, Butylthio, 1-Methylpropylthio, 2-Methylpropylthio und 1,1-Dimethylethylthio;
- C₁-C₆-Alkylthio: C₁-C₄-Alkylthio, wie voranstehend genannt, sowie z. B. Pentylthio, 1-Methylbutylthio, 2-Methylbutylthio, 3-Methylbutylthio, 2,2-Dimethylpropylthio, 1-Ethylpropylthio, Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio oder 1-Ethyl-2-methylpropylthio;
- C₁-C₄-Halogenalkylthio : einen C₁-C₄-Alkylthiorest, wie voranstehend genannt, der partiell oder vollständig durch Fluor, Chlor, Brom und/oder Iod substituiert ist, also z. B. Fluormethylthio, Difluormethylthio, Trifluormethylthio, Chlordifluormethylthio, Bromdifluormethylthio, 2-Fluorethylthio, 2-Chlorethylthio, 2-Bromethylthio, 2-Iodethylthio, 2,2-Difluorethylthio, 2,2,2-Trifluorethylthio, 2,2,2-Trichlorethylthio, 2-Chlor-2-fluorethylthio, 2-Chlor-2,2-difluorethylthio, 2,2-Dichlor-2-fluorethylthio, Pentafluorethylthio, 2-Fluorpropylthio, 3-Fluorpropylthio, 2-Chlorpropylthio, 3-Chlorpropylthio, 2-Brompropylthio, 3-Brompropylthio, 2,2-Difluorpropylthio, 2,3-Difluorpropylthio, 2,3-Dichlorpropylthio, 3,3,3-Trifluorpropylthio, 3,3,3-Trichlorpropylthio, 2,2,3,3,3-Pentafluorpropylthio, Heptafluorpropylthio, 1-(Fluormethyl)-2-fluorethylthio, 1-(Chlormethyl)-2-chlorethylthio, 1-(Brommethyl)-2-bromethylthio, 4-Fluorbutylthio, 4-Chlorbutylthio, 4-Brombutylthio und Nonafluorbutylthio;
- C₁-C₆-Halogenalkylthio: C₁-C₄-Halogenalkylthio, wie voranstehend genannt, sowie z. B. 5-Fluorpentylthio, 5-Chlorpentylthio, 5-Brompentylthio, 5-Iodpentylthio, Undecafluorpentylthio, 6-Fluorhexylthio, 6-Chlorhexylthio, 6-Bromhexylthio, 6-Iodhexylthio oder Dodecafluorhexylthio;
- C₁-C₆-Alkylsulfinyl (C₁-C₆-Alkyl-S(=O)-): z. B. Methylsulfinyl, Ethylsulfinyl, Propylsulfinyl, 1-Methylethylsulfinyl, Butylsulfinyl, 1-Methylpropylsulfinyl, 2-Methylpropylsulfinyl, 1,1-Dimethylethylsulfinyl, Pentylsulfinyl, 1-Methylbutylsulfinyl, 2-Methylbutylsulfinyl, 3-Methylbutylsulfinyl, 2,2-Dimethylpropylsulfinyl, 1-Ethylpropylsulfinyl, 1,1-Dimethylpropylsulfinyl, 1,2-Dimethylpropylsulfinyl, Hexylsulfinyl, 1-Methylpentylsulfinyl, 2-Methylpentylsulfinyl, 3-Methylpentylsulfinyl, 4-Methylpentylsulfinyl, 1,1-Dimethylbutylsulfinyl, 1,2-Dimethylbutylsulfinyl, 1,3-Dimethylbutylsulfinyl, 2,2-Dimethylbutylsulfinyl, 2,3-Dimethylbutylsulfinyl, 3,3-Dimethylbutylsulfinyl, 1-Ethylbutylsulfinyl, 2-Ethylbutylsulfinyl, 1,1,2-Trimethylpropylsulfinyl, 1,2,2-Trimethylpropylsulfinyl, 1-Ethyl-1-methylpropylsulfinyl oder 1-Ethyl-2-methylpropylsulfinyl;
- C₁-C₆-Halogenalkylsulfinyl: C₁-C₆-Alkylsulfinylrest, wie voranstehend genannt, der partiell oder vollständig durch Fluor, Chlor, Brom und/oder Iod substituiert ist, also z. B. Fluormethylsulfinyl, Difluormethylsulfinyl, Trifluormethylsulfinyl, Chlordifluormethylsulfinyl, Bromdifluormethylsulfinyl, 2-Fluorethylsulfinyl, 2-Chlorethylsulfinyl, 2-Bromethylsulfinyl, 2-Iodethylsulfinyl, 2,2-Difluorethylsulfinyl, 2,2,2-Trifluorethylsulfinyl, 2,2,2-Trichlorethylsulfinyl, 2-Chlor-2-fluorethylsulfinyl, 2-Chlor-2,2-difluorethylsulfinyl, 2,2-Dichlor-2-fluorethylsulfinyl, Pentafluorethylsulfinyl, 2-Fluorpropylsulfinyl, 3-Fluorpropylsulfinyl, 2-Chlorpropylsulfinyl, 3-Chlorpropylsulfinyl, 2-Brompropylsulfinyl, 3-Brompropylsulfinyl, 2,2-Difluorpropylsulfinyl, 2,3-Difluorpropylsulfinyl, 2,3-Dichlorpropylsulfinyl, 3,3,3-Trifluorpropylsulfinyl, 3,3,3-Trichlorpropylsulfinyl, 2,2,3,3,3-Pentafluorpropylsulfinyl, Heptafluorpropylsulfinyl, 1-(Fluormethyl)-2-fluorethylsulfinyl, 1-(Chlormethyl)-2-chlorethylsulfinyl, 1-(Brommethyl)-2-bromethylsulfinyl, 4-Fluorbutylsulfinyl, 4-Chlorbutylsulfinyl, 4-Brombutylsulfinyl, Nonafluorbutylsulfinyl, 5-Fluorpentylsulfinyl, 5-Chlorpentylsulfinyl, 5-Brompentylsulfinyl, 5-Iodpentylsulfinyl, Undecafluorpentylsulfinyl, 6-Fluorhexylsulfinyl, 6-Chlorhexylsulfinyl, 6-Bromhexylsulfinyl, 6-Iodhexylsulfinyl oder Dodecafluorhexylsulfinyl;
- C₁-C₄-Alkylsulfonyl (C₁-C₄-Alkyl-S(=O)₂-), sowie die Alkylsulfonylteile von C₁-C₄-Alkylsulfonyloxy: z. B. Methylsulfonyl, Ethylsulfonyl, Propylsulfonyl, 1-Methylethylsulfonyl, Butylsulfonyl, 1-Methylpropylsulfonyl, 2-Methylpropylsulfonyl und 1,1-Dimethylethylsulfonyl;
- C₁-C₆-Alkylsulfonyl, sowie die Alkylsulfonylteile von C₁-C₆-Alkylsulfonyloxy: einen C₁-C₄-Alkylsulfonylrest, wie voranstehend genannt, sowie z. B. Pentylsulfonyl, 1-Methylbutylsulfonyl, 2-Methylbutylsulfonyl, 3-Methylbutylsulfonyl, 1,1-Dimethylpropylsulfonyl, 1,2-Dimethylpropylsulfonyl, 2,2-Dimethylpropylsulfonyl, 1-Ethylpropylsulfonyl, Hexylsulfonyl, 1-Methylpentylsulfonyl, 2-Methylpentylsulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl und 1-Ethyl-2-methylpropylsulfonyl ;
- C₁-C₆-Halogenalkylsulfonyl : einen C₁-C₆-Alkylsulfonylrest, wie voranstehend genannt, der partiell oder vollständig durch Fluor, Chlor, Brom und/oder Iod substituiert ist, also z. B. Fluormethylsulfonyl, Difluormethylsulfonyl, Trifluormethylsulfonyl, Chlordifluormethylsulfonyl, Bromdifluormethylsulfonyl, 2-Fluorethylsulfonyl, 2-Chlorethylsulfonyl, 2-Bromethylsulfonyl, 2-Iodethylsulfonyl, 2,2-Difluorethylsulfonyl, 2,2,2-Trifluorethylsulfonyl, 2-Chlor-2-fluorethylsulfonyl, 2-Chlor-2,2-difluorethylsulfonyl, 2,2-Dichlor-2-fluorethylsulfonyl, 2,2,2-Trichlorethylsulfonyl, Pentafluorethylsulfonyl, 2-Fluorpropylsulfonyl, 3-Fluorpropylsulfonyl, 2-Chlorpropylsulfonyl, 3-Chlorpropylsulfonyl, 2-Brompropylsulfonyl, 3-Brompropylsulfonyl, 2,2-Difluorpropylsulfonyl, 2,3-Difluorpropylsulfonyl, 2,3-Dichlorpropylsulfonyl, 3,3,3-Trifluorpropylsulfonyl, 3,3,3-Trichlorpropylsulfonyl, 2,2,3,3,3-Pentafluorpropylsulfonyl, Heptafluorpropylsulfonyl, 1-(Fluormethyl)-2-fluorethylsulfonyl, 1-(Chlormethyl)-2-chlorethylsulfonyl, 1-(Brommethyl)-2-bromethylsulfonyl, 4-Fluorbutylsulfonyl, 4-Chlorbutylsulfonyl, 4-Brombutylsulfonyl, Nonafluorbutylsulfonyl, 5-Fluorpentylsulfonyl, 5-Chlorpentylsulfonyl, 5-Brompentylsulfonyl, 5-Iodpentylsulfonyl, 6-Fluorhexylsulfonyl, 6-Bromhexylsulfonyl, 6-Iodhexylsulfonyl oder Dodecafluorhexylsulfonyl;
- C₃-C₆-Alkenyloxy: z. B. Prop-1-en-1-yloxy, Prop-2-en-1-yloxy, 1-Methylethenyloxy, Buten-1-yloxy, Buten-2-yloxy, Buten-3-yl-oxy, 1-Methyl-prop-1-en-1-yloxy, 2-Methyl-prop-1-en-1-yloxy, 1-Methyl-prop-2-en-1-yloxy, 2-Methyl-prop-2-en-1-yloxy, Penten-1-yloxy, Penten-2-yloxy, Penten-3-yloxy, Penten-4-yloxy, 1-Methyl-but-1-en-1-yloxy, 2-Methyl-but-1-en-1-yloxy, 3-Methyl-but-1-en-1-yloxy, 1-Methyl-but-2-en-1-yloxy, 2-Methyl-but-2-en-1-yloxy, 3-Methyl-but-2-en-1-yloxy, 1-Methyl-but-3-en-1-yloxy, 2-Methyl-but-3-en-1-yloxy, 3-Methyl-but-3-en-1-yloxy, 1,1-Dimethyl-prop-2-en-1-yloxy; 1,2-Dimethyl-prop-1-en-1-yloxy, 1,2-Dimethyl-prop-2-en-1-yl-oxy, 1-Ethyl-prop-1-en-2-yloxy, 1-Ethyl-prop-2-en-1-yloxy, Hex-1-en-1-yloxy, Hex-2-en-1-yloxy, Hex-3-en-1-yloxy, Hex-4-en-1-yloxy, Hex-5-en-1-yloxy, 1-Methyl-pent-1-en-l-yl-oxy, 2-Methyl-pent-1-en-1-yloxy, 3-Methyl-pent-1-en-1-yloxy, 4-Methyl-pent-1-en-1-yloxy, 1-Methyl-pent-2-en-1-yloxy, 2-Methyl-pent-2-en-1-yloxy, 3-Methyl-pent-2-en-1-yloxy, 4-Methyl-pent-2-en-1-yloxy, 1-Methyl-pent-3-en-1-yloxy, 2-Methyl-pent-3-en-1-yloxy, 3-Methyl-pent-3-en-1-yloxy, 4-Methyl-pent-3-en-1-yloxy, 1-Methyl-pent-4-en-1-yloxy, 2-Methyl-pent-4-en-1-yloxy, 3-Methyl-pent-4-en-1-yloxy, 4-Methyl-pent-4-en-1-yloxy, 1,1-Dimethyl-but-2-en-1-yloxy, 1,1-Dimethyl-but-3-en-1-yloxy, 1,2-Dimethyl-but-1-en-1-yloxy, 1,2-Dimethyl-but-2-en-1-yloxy, 1,2-Dimethyl-but-3-en-1-yloxy, 1,3-Dimethyl-but-1-en-1-yloxy, 1,3-Dimethyl-but-2-en-1-yloxy, 1,3-Dimethyl-but-3-en-1-yloxy, 2,2-Dimethyl-but-3-en-1-yloxy, 2,3-Dimethyl-but-1-en-1-yloxy, 2,3-Dimethyl-but-2-en-1-yloxy, 2,3-Dimethyl-but-3-en-1-yloxy, 3,3-Dimethyl-but-1-en-1-yloxy, 3,3-Dimethyl-but-2-en-1-yloxy, 1-Ethyl-but-1-en-1-yloxy, 1-Ethyl-but-2-en-1-yloxy, 1-Ethyl-but-3-en-1-yloxy, 2-Ethyl-but-1-en-1-yloxy, 2-Ethyl-but-2-en-1-yloxy, 2-Ethyl-but-3-en-1-yloxy, 1,1,2-Trimethyl-prop-2-en-1-yloxy, 1-Ethyl-1-methyl-prop-2-en-1-yloxy, 1-Ethyl-2-methyl-prop-1-en-1-yloxy und 1-Ethyl-2-methyl-prop-2-en-1-yloxy;
- C₃-C₆-Alkenyl : Prop-1-en-1-yl, Prop-2-en-1-yl, 1-Methylethenyl, Buten-1-yl, Buten-2-yl, Buten-3-yl, 1-Methyl-prop-1-en-1-yl, 2-Methyl-prop-1-en-1-yl, 1-Methyl-prop-2-en-1-yl, 2-Methyl-prop-2-en-1-yl, Penten-1-yl, Penten-2-yl, Penten-3-yl, Penten-4-yl, 1-Methyl-but-1-en-1-yl, 2-Methyl-but-1-en-1-yl, 3-Methyl-but-1-en-1-yl, 1-Methyl-but-2-en-1-yl, 2-Methyl-but-2-en-1-yl, 3-Methyl-but-2-en-1-yl, 1-Methyl-but-3-en-1-yl, 2-Methyl-but-3-en-1-yl, 3-Methyl-but-3-en-1-yl, 1,1-Dimethyl-prop-2-en-1-yl, 1,2-Dimethyl-prop-1-en-1-yl, 1,2-Dimethyl-prop-2-en-1-yl, 1-Ethyl-prop-1-en-2-yl, 1-Ethyl-prop-2-en-1-yl, Hex-1-en-1-yl, Hex-2-en-1-yl, Hex-3-en-1-yl, Hex-4-en-1-yl, Hex-5-en-1-yl, 1-Methyl-pent-1-en-1-yl, 2-Methyl-pent-1-en-1-yl, 3-Methyl-pent-1-en-1-yl, 4-Methyl-pent-1-en-1-yl, 1-Methyl-pent-2-en-1-yl, 2-Methyl-pent-2-en-1-yl, 3-Methyl-pent-2-en-1-yl, 4-Methyl-pent-2-en-1-yl, 1-Methyl-pent-3-en-1-yl, 2-Methyl-pent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethyl-but-1-en-1-yl, 1,2-Dimethyl-but-2-en-1-yl, 1,2-Dimethyl-but-3-en-1-yl, 1,3-Dimethyl-but-1-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-1-en-1-yl, 2,3-Dimethyl-but-2-en-1-yl, 2,3-Dimethyl-but-3-en-1-yl, 3,3-Dimethyl-but-1-en-1-yl, 3,3-Dimethyl-but-2-en-1-yl, 1-Ethyl-but-1-en-1-yl, 1-Ethyl-but-2-en-1-yl, 1-Ethyl-but-3-en-1-yl, 2-Ethyl-but-1-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl, 1-Ethyl-2-methyl-prop-1-en-1-yl und 1-Ethyl-2-methyl-prop-2-en-1-yl;
- C₁-C₄-Alkandiyl: z. B. Methandiyl, 1,2-Ethandiyl, 1,3-Propandiyl und 1,4-Butandiyl.

Die Phenylringe der Reste Phenylalkyl-, Phenylcarbonylalkyl-, Phenylalkoxy-, Phenylcarbonylalkoxy-, Phenylsulfonyl-, Phenylsulfonyloxy-, Phenylcarbonyl- und Phenylcarbonyloxy- sind vorzugsweise unsubstituiert oder tragen ein, zwei oder drei Halogenatome und/oder eine Nitrogruppe, eine Cyanogruppe, eine oder zwei Methyl-, Trifluormethyl-, Methoxy- oder Trifluormethoxygruppen.

In der Formel I steht vorzugsweise
- R¹: für C₁-C₆-Alkyl, C₁-C₆-Halogenalkyl, C₁-C₆-Alkoxy oder Halogen;
insbesondere C₁-C₄-Alkyl, vorzugsweise Methyl, Ethyl, n-Propyl oder i-Propyl; oder Halogen, vorzugsweise Fluor, Chlor oder Brom;
besonders bevorzugt Methyl oder Chlor;
am stärksten bevorzugt Methyl;
- R²: für C₁-C₆-Halogenalkyl, C₁-C₆-Alkylsulfonyl, Halogen oder Nitro;
insbesondere C₁-C₄-Halogenalkyl, vorzugsweise Difluormethyl oder Trifluormethyl; C₁-C₄-Alkylsulfonyl, vorzugsweise Methylsulfonyl oder Ethylsulfonyl; oder Halogen, vorzugsweise Fluor oder Chlor;
besonders bevorzugt C₁-C₄-Alkylsulfonyl, wobei Methylsulfonyl am stärksten bevorzugt ist;
- R³: für Wasserstoff, C₁-C₄-Alkyl oder Halogen; insbesondere Wasserstoff, Chlor oder Methyl; besonders bevorzugt Wasserstoff;
- R⁴: für Wasserstoff, C₁-C₄-Alkyl oder C₁-C₄-Halogenalkyl; insbesondere für Wasserstoff, Methyl, Ethyl, Chlormethyl oder Brommethyl;
- R⁵: für Wasserstoff oder C₁-C₄-Alkyl ; insbesondere für Wasserstoff oder Methyl;
besonders bevorzugt für Wasserstoff; oder
- R⁴,R⁵: gemeinsam für eine C₁-C₄-Alkandiylgruppe stehen; insbesondere für eine Methandiylgruppe steht;
- R⁶: für Hydroxy, C₁-C₆-Alkoxy, C₁-C₆-Alkylsulfonyloxy, C₁-C₆-Alkylcarbonyloxy, Phenyl-C₁-C₂-alkoxy, Phenylcarbonyl-C₁-C₂-alkoxy, Phenylsulfonyloxy, Phenylcarbonyloxy, wobei der Phenylrest der vier letztgenannten Substituenten partiell oder vollständig halogeniert sein kann und/oder eine bis drei der folgenden Gruppen tragen kann: Nitro, Cyano, C₁-C₄-Alkyl, C₁-C₄-Halogenalkyl, C₁-C₄-Alkoxy, oder C₁-C₄-Halogenalkoxy ;
insbesondere Hydroxy, Phenyl-C₁-C₂-alkoxy, Phenylcarbonyl-C₁-C₂-alkoxy, Phenylsulfonyloxy, Phenylcarbonyloxy, wobei der Phenylrest der vier letztgenannten Substituenten partiell oder vollständig halogeniert sein kann oder eine bis drei der folgenden Gruppen tragen kann: Nitro, Cyano, C₁-C₄-Alkyl, C₁-C₄-Halogenalkyl, C₁-C₄-Alkoxy, oder C₁-C₄-Halogenalkoxy;
besonders bevorzugt Hydroxy;
- R⁷: für Wasserstoff, C₁-C₄-Alkyl oder Cyclopropyl; insbesondere C₁-C₄-Alkyl, vorzugsweise Methyl, Ethyl, i-Propyl, i-Butyl, s-Butyl oder t-Butyl; oder Cyclopropyl;
- R⁸: für Wasserstoff oder C₁-C₄-Alkyl; insbesondere Wasserstoff, Methyl oder Ethyl; besonders bevorzugt Wasserstoff oder Methyl.

Besonders bevorzugt sind Cyclopropyl-anellierte 3-(4,5-Dihydroisoxazol-3-yl)-substituierte Benzoylpyrazole der Formel I, worin
- R¹: für C₁-C₆-Alkyl oder Halogen ;
- R²: für C₁-C₆-Halogenalkyl, C₁-C₆-Alkylsulfonyl oder Halogen;
- R³: für Wasserstoff, C₁-C₄-Alkyl oder Halogen;
- R⁷: für C₁-C₄-Alkyl oder Cyclopropyl;
- R⁸: für Wasserstoff oder C₁-C₄-Alkyl steht.

Besonders bevorzugt sind weiterhin Cyclopropyl-anellierte 3-(4,5-Dihydroisoxazol-3-yl)-substituierte Benzoylpyrazole der Formel I, worin
- R¹: für Methyl oder Chlor;
- R²: für C₁-C₄-Alkylsulfonyl;
- R³: für Wasserstoff, Methyl oder Chlor;
- R⁴: für Wasserstoff, Methyl, Ethyl, Chlormethyl oder Brommethyl; oder
- R⁵: für Wasserstoff oder Methyl; insbesondere für Wasserstoff;
- R⁴,R⁵: gemeinsam eine Methandiylgruppe bilden;
- R⁶: für Hydroxy;
- R⁷: für C₁-C₄-Alkyl oder Cyclopropyl;
- R⁸: für Wasserstoff oder C₁-C₄-Alkyl steht.

Außerordentlich bevorzugt sind die Verbindungen der Formel Ia1 (≡I mit R³, R⁸ = H; R⁶ = OH; R⁷ = CH₃), insbesondere die Verbindungen Ia1.1 bis Ia1.77 der Tabelle 1, wobei die Definitionen der Reste R¹ bis R⁸ nicht nur in Kombination miteinander, sondern auch jeweils für sich allein betrachtet für die erfindungsgemäßen Verbindungen eine besondere Bedeutung haben.

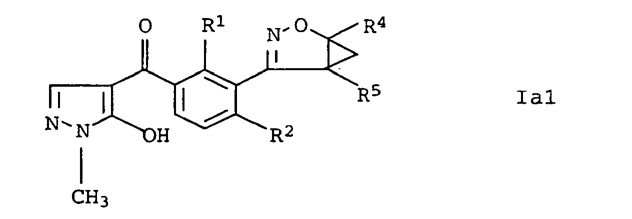

**Tabelle 1:**

| Nr. | R¹ | R² | R⁴ | R⁵ |
|---|---|---|---|---|
| Ia1.1 | Cl | SO₂CH₃ | H | H |
| Ia1.2 | Cl | SO₂CH₃ | CH₃ | H |
| Ia1.3 | Cl | SO₂CH₃ | CH₃ | CH₃ |
| Ia1.4 | Cl | SO₂CH₃ | CH₂CH₃ | H |
| Ia1.5 | Cl | SO₂CH₃ | CH₂Cl | H |
| Ia1.6 | Cl | SO₂CH₃ | CH₂F | H |
| Ia1.7 | Cl | SO₂CH₃ | CH₂Br | H |
| Ia1.8 | Cl | SO₂CH₃ | CF₃ | H |
| Ia1.9 | Cl | SO₂CH₃ | CHClCH₃ | H |
| Ia1.10 | Cl | SO₂CH₃ | CHFCH₃ | H |
| Ia1.11 | Cl | SO₂CH₃ | CH₂ | |
| Ia1.12 | CH₃ | SO₂CH₃ | H | H |
| Ia1.13 | CH₃ | SO₂CH₃ | CH₃ | H |
| Ia1.14 | CH₃ | SO₂CH₃ | CH₃ | CH₃ |
| Ia1.15 | CH₃ | SO₂CH₃ | CH₂CH₃ | H |
| Ia1.16 | CH₃ | SO₂CH₃ | CH₂Cl | H |
| Ia1.17 | CH₃ | SO₂CH₃ | CH₂F | H |
| Ia1.18 | CH₃ | SO₂CH₃ | CH₂Br | H |
| Ia1.19 | CH₃ | SO₂CH₃ | CF₃ | H |
| Ia1.20 | CH₃ | SO₂CH₃ | CHClCH₃ | H |
| Ia1.21 | CH₃ | SO₂CH₃ | CHFCH₃ | H |
| Ia1.22 | CH₃ | SO₂CH₃ | CH₂ | |
| Ia1.23 | Cl | CF₃ | H | H |
| Ia1.24 | Cl | CF₃ | CH₃ | H |
| Ia1.25 | Cl | CF₃ | CH₃ | CH₃ |
| Ia1.26 | Cl | CF₃ | CH₂CH₃ | H |
| Ia1.27 | Cl | CF₃ | CH₂Cl | H |
| Ia1.28 | Cl | CF₃ | CH₂F | H |
| Ia1.29 | Cl | CF₃ | CH₂Br | H |
| Ia1.30 | Cl | CF₃ | CF₃ | H |
| Ia1.31 | Cl | CF₃ | CHClCH₃ | H |
| Ial.32 | Cl | CF₃ | CHFCH₃ | H |
| Ia1.33 | Cl | CF₃ | CH₂ | |
| Ia1.34 | CH₃ | CF₃ | H | H |
| Ia1.35 | CH₃ | CF₃ | CH₃ | H |
| Ia1.36 | CH₃ | CF₃ | CH₃ | CH₃ |
| Ial.37 | CH₃ | CF₃ | CH₂CH₃ | H |
| Ial.38 | CH₃ | CF₃ | CH₂C1 | H |
| Ial.39 | CH₃ | CF₃ | CH₂F | H |
| Ial.40 | CH₃ | CF₃ | CH₂Br | H |
| Ial.41 | CH₃ | CF₃ | CF₃ | H |
| Ial.42 | CH₃ | CF₃ | CHC1CH₃ | H |
| Ia1.43 | CH₃ | CF₃ | CHFCH₃ | H |
| Ial.44 | CH₃ | CF₃ | CH₂ | |
| Ial.45 | CH₂CH₃ | SO₂CH₃ | H | H |
| Ia1.46 | CH₂CH₃ | SO₂CH₃ | CH₃ | H |
| Ia1.47 | CH₂CH₃ | SO₂CH₃ | CH₃ | CH₃ |
| Ia1.48 | CH₂CH₃ | SO₂CH₃ | CH₂CH₃ | H |
| Ia1.49 | CH₂CH₃ | SO₂CH₃ | CH₂Cl | H |
| Ia1.50 | CH₂CH₃ | SO₂CH₃ | CH₂F | H |
| Ia1.51 | CH₂CH₃ | SO₂CH₃ | CH₂Br | H |
| Ia1.52 | CH₂CH₃ | SO₂CH₃ | CF₃ | H |
| Ia1.53 | CH₂CH₃ | SO₂CH₃ | CHClCH₃ | H |
| Ia1.54 | CH₂CH₃ | SO₂CH₃ | CHFCH₃ | H |
| Ia1.55 | CH₂CH₃ | SO₂CH₃ | CH₂ | |
| Ia1.56 | CH₃ | SO₂CH₂CH₃ | H | H |
| Ia1.57 | CH₃ | SO₂CH₂CH₃ | CH₃ | H |
| Ia1.58 | CH₃ | SO₂CH₂CH₃ | CH₃ | CH₃ |
| Ia1.59 | CH₃ | SO₂CH₂CH₃ | CH₂CH₃ | H |
| Ia1.60 | CH₃ | SO₂CH₂CH₃ | CH₂C1 | H |
| Ia1.61 | CH₃ | SO₂CH₂CH₃ | CH₂F | H |
| Ia1.62 | CH₃ | SO₂CH₂CH₃ | CH₂Br | H |
| Ia1.63 | CH₃ | SO₂CH₂CH₃ | CF₃ | H |
| Ia1.64 | CH₃ | SO₂CH₂CH₃ | CHC1CH₃ | H |
| Ia1.65 | CH₃ | SO₂CH₂CH₃ | CHFCH₃ | H |
| Ia1.66 | CH₃ | SO₂CH₂CH₃ | CH₂ | |
| Ia1.67 | Cl | SO₂CH₂CH₃ | H | H |
| Ia1.68 | Cl | SO₂CH₂CH₃ | CH₃ | H |
| Ia1.69 | Cl | SO₂CH₂CH₃ | CH₃ | CH₃ |
| Ia1.70 | Cl | SO₂CH₂CH₃ | CH₂CH₃ | H |
| Ia1.71 | Cl | SO₂CH₂CH₃ | CH₂Cl | H |
| Ia1.72 | Cl | SO₂CH₂CH₃ | CH₂F | H |
| Ia1.73 | Cl | SO₂CH₂CH₃ | CH₂Br | H |
| Ia1.74 | Cl | SO₂CH₂CH₃ | CF₃ | H |
| Ia1.75 | Cl | SO₂CH₂CH₃ | CHClCH₃ | H |
| Ia1.76 | Cl | SO₂CH₂CH₃ | CHFCH₃ | H |
| Ia1.77 | Cl | SO₂CH₂CH₃ | CH₂ | |

Ebenso außerordentlichst bevorzugt sind die Verbindungen der Formel Ia2, insbesondere die Verbindungen Ia2.1 bis Ia2.77, die sich von den entsprechenden Verbindungen Ia1.1 bis Ia1.77 dadurch unterscheiden, dass R⁷ für Ethyl steht.

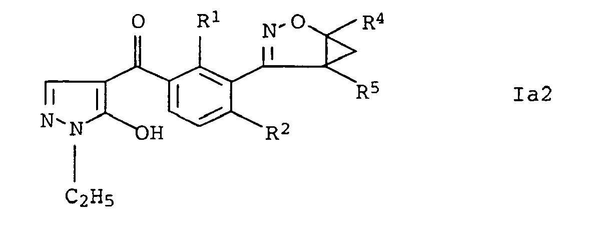

Ebenso außerordentlichst bevorzugt sind die Verbindungen der Formel Ia3, insbesondere die Verbindungen Ia3.1 bis Ia3.77, die sich von den entsprechenden Verbindungen Ia1.1 bis Ia1.77 dadurch unterscheiden, dass R⁷ für i-Propyl steht.

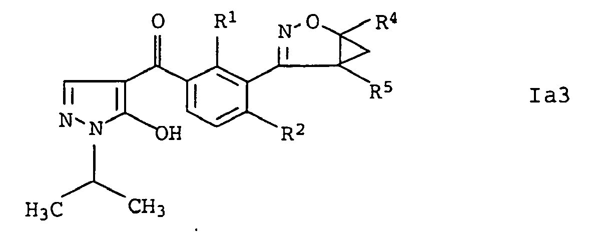

Ebenso außerordentlichst bevorzugt sind die Verbindungen der Formel Ia4, insbesondere die Verbindungen Ia4.1 bis Ia4.77, die sich von den entsprechenden Verbindungen Ia1.1 bis Ia1.77 dadurch unterscheiden, dass R⁷ für t-Butyl steht.

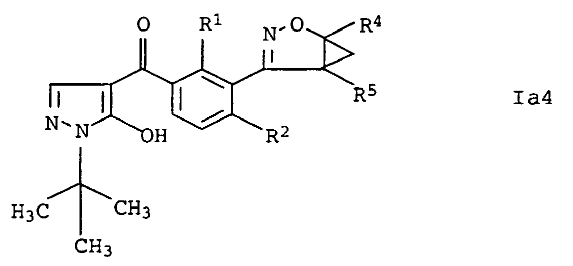

Ebenso außerordentlichst bevorzugt sind die Verbindungen der Formel Ia5, insbesondere die Verbindungen Ia5.1 bis Ia5.77, die sich von den entsprechenden Verbindungen Ia1.1 bis Ia1.77 dadurch unterscheiden, dass R⁷ für Cyclopropyl steht.

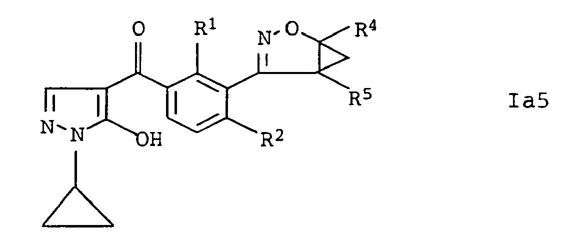

Ebenso außerordentlichst bevorzugt sind die Verbindungen der Formel Ia6, insbesondere die Verbindungen Ia6.1 bis Ia6.77, die sich von den entsprechenden Verbindungen Ial.1 bis Ia1.77 dadurch unterscheiden, dass R⁸ für Methyl steht.

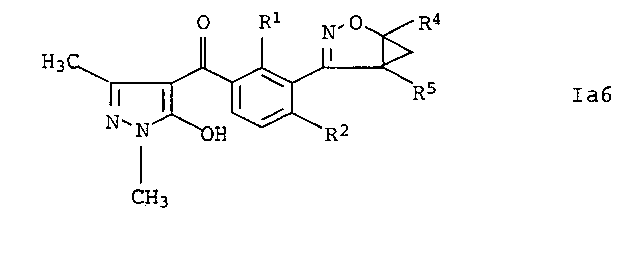

Ebenso außerordentlichst bevorzugt sind die Verbindungen der Formel Ia7, insbesondere die Verbindungen Ia7.1 bis Ia7.77, die sich von den entsprechenden Verbindungen Ia1.1 bis Ia1.77 dadurch unterscheiden, dass R⁸ für Methyl und R⁷ für Ethyl stehen.

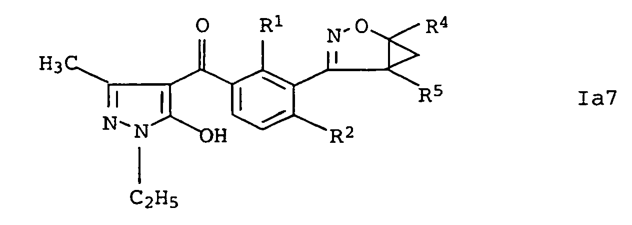

Ebenso außerordentlichst bevorzugt sind die Verbindungen der Formel Ia8, insbesondere die Verbindungen Ia8.1 bis Ia8.77, die sich von den entsprechenden Verbindungen Ia1.1 bis Ia1.77 dadurch unterscheiden, dass R⁸ für Methyl und R⁷ für i-Propyl stehen.

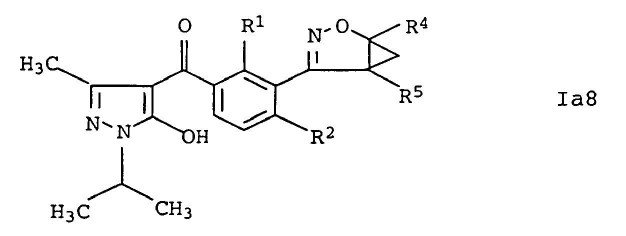

Ebenso außerordentlichst bevorzugt sind die Verbindungen der Formel Ia9, insbesondere die Verbindungen Ia9.1 bis Ia9.77, die sich von den entsprechenden Verbindungen Ia1.1 bis Ia1.77 dadurch unterscheiden, dass R⁸ für Methyl und R⁷ für t-Butyl stehen.

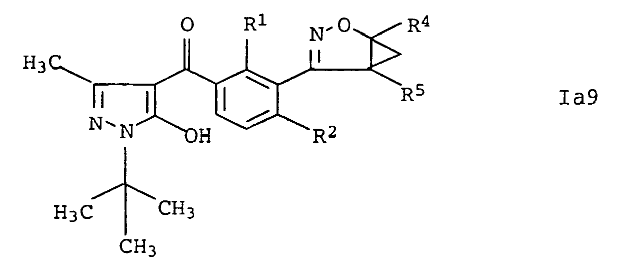

Ebenso außerordentlichst bevorzugt sind die Verbindungen der Formel Ia10, insbesondere die Verbindungen Ia10.1 bis Ia10.77, die sich von den entsprechenden Verbindungen Ia1.1 bis Ial.77 dadurch unterscheiden, dass R⁸ für Methyl und R⁷ für Cyclopropyl stehen.

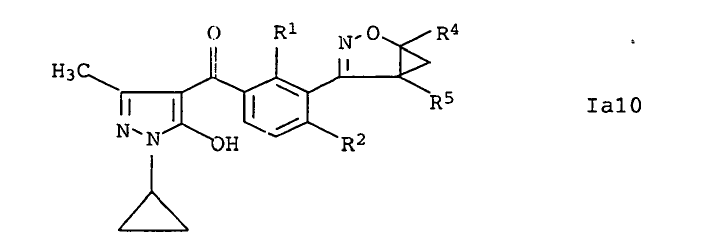

Ebenso außerordentlichst bevorzugt sind die Verbindungen der Formel Ia11, insbesondere die Verbindungen Ia11.1 bis Ia11.77, die sich von den entsprechenden Verbindungen Ia1.1 bis Ia1.77 dadurch unterscheiden, dass R⁶ für Phenylcarbonyloxy steht.

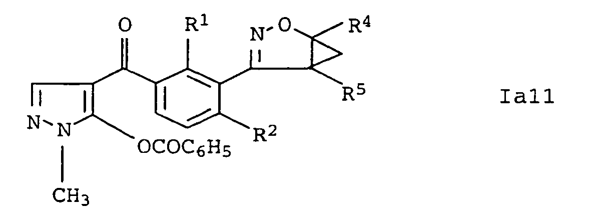

Ebenso außerordentlichst bevorzugt sind die Verbindungen der Formel Ia12, insbesondere die Verbindungen Ia12.1 bis Ia12.77, die sich von den entsprechenden Verbindungen Ia1.1 bis Ia1.77 dadurch unterscheiden, dass R⁶ für Phenylcarbonyloxy und R⁷ für Ethyl stehen.

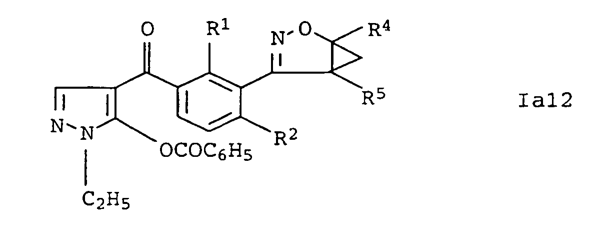

Ebenso außerordentlichst bevorzugt sind die Verbindungen der Formel Ia13, insbesondere die Verbindungen Ia13.1 bis Ia13.77, die sich von den entsprechenden Verbindungen Ia1.1 bis Ia1.77 dadurch unterscheiden, dass R⁶ für Phenylcarbonyloxy und R⁷ für i-Propyl stehen.

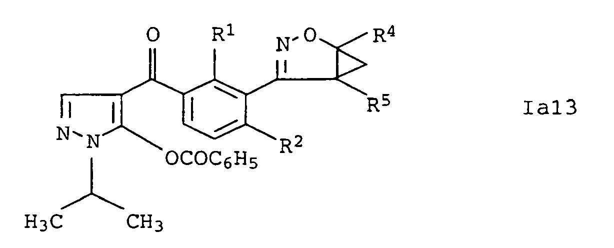

Ebenso außerordentlichst bevorzugt sind die Verbindungen der Formel Ia14, insbesondere die Verbindungen Ia14.1 bis Ia14.77, die sich von den entsprechenden Verbindungen Ia1.1 bis Ia1.77 dadurch unterscheiden, dass R⁶ für Phenylcarbonyloxy und R⁷ für t-Butyl stehen.

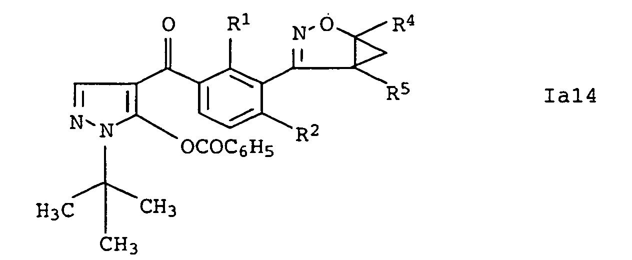

Ebenso außerordentlichst bevorzugt sind die Verbindungen der Formel Ia15, insbesondere die Verbindungen Ia15.1 bis Ia15.77, die sich von den entsprechenden Verbindungen Ia1.1 bis Ia1.77 dadurch unterscheiden, dass R⁶ für 3-Fluorphenylcarbonyloxy steht.

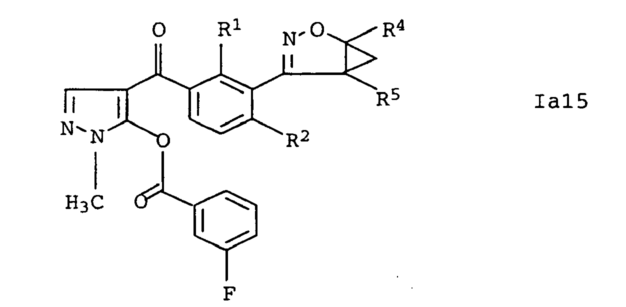

Ebenso außerordentlichst bevorzugt sind die Verbindungen der Formel Ia16, insbesondere die Verbindungen Ia16.1 bis Ia16.77, die sich von den entsprechenden Verbindungen Ia1.1 bis Ia1.77 dadurch unterscheiden, dass R⁶ für 3-Fluorphenylcarbonyloxy und R⁷ für Ethyl stehen.

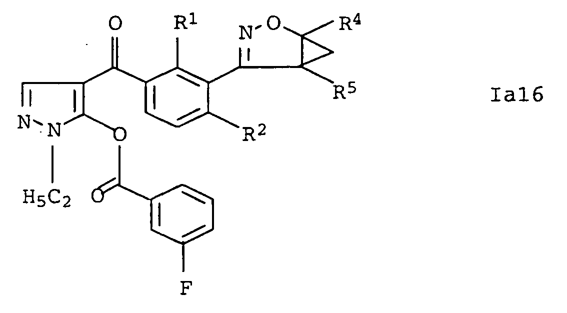

Ebenso außerordentlichst bevorzugt sind die Verbindungen der Formel Ia17, insbesondere die Verbindungen Ia17.1 bis Ia17.77, die sich von den entsprechenden Verbindungen Ia1.1 bis Ia1.77 dadurch unterscheiden, dass R⁶ für 3-Fluorphenylcarbonyloxy und R⁷ für i-Propyl stehen.

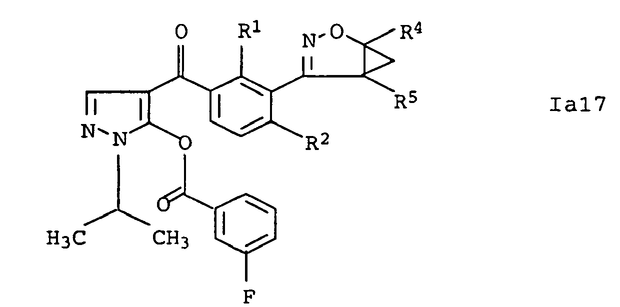

Ebenso außerordentlichst bevorzugt sind die Verbindungen der Formel Ia18, insbesondere die Verbindungen Ia18.1 bis Ia18.77, die sich von den entsprechenden Verbindungen Ia1.1 bis Ia1.77 dadurch unterscheiden, dass R⁶ für 3-Fluorphenylcarbonyloxy und R⁷ für t-Butyl stehen.

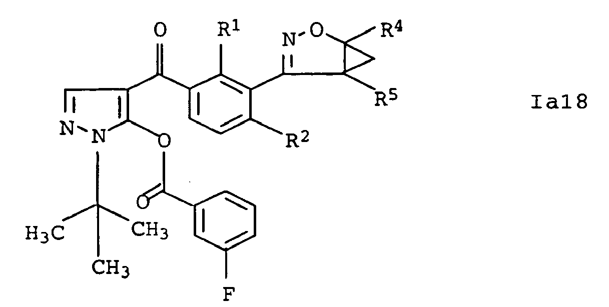

Ebenso außerordentlichst bevorzugt sind die Verbindungen der Formel Ia19, insbesondere die Verbindungen Ia19.1 bis Ia19.77, die sich von den entsprechenden Verbindungen Ia1.1 bis Ia1.77 dadurch unterscheiden, dass R⁶ für 3-Trifluormethylphenylcarbonyloxy steht.

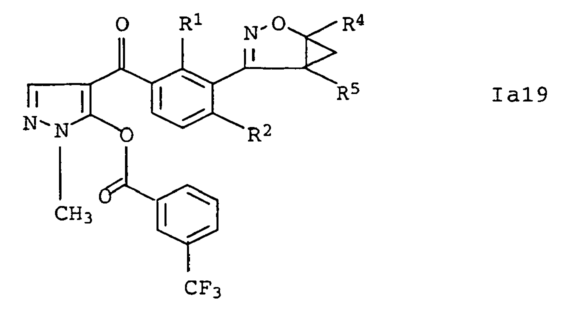

Ebenso außerordentlichst bevorzugt sind die Verbindungen der Formel Ia20, insbesondere die Verbindungen Ia20.1 bis Ia20.77, die sich von den entsprechenden Verbindungen Ia1.1 bis Ia1.77 dadurch unterscheiden, dass R⁶ für 3-Trifluormethylphenylcarbonyloxy und R⁷ für Ethyl stehen.

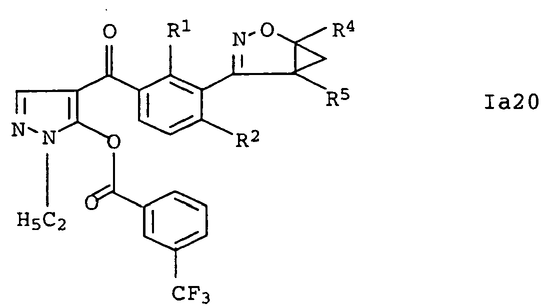

Ebenso außerordentlichst bevorzugt sind die Verbindungen der Formel Ia21, insbesondere die Verbindungen Ia21.1 bis Ia21.77, die sich von den entsprechenden Verbindungen Ia1.1 bis Ia1.77 dadurch unterscheiden, dass R⁶ für 3-Trifluormethylphenylcarbonyloxy und R⁷ für i-Propyl stehen.

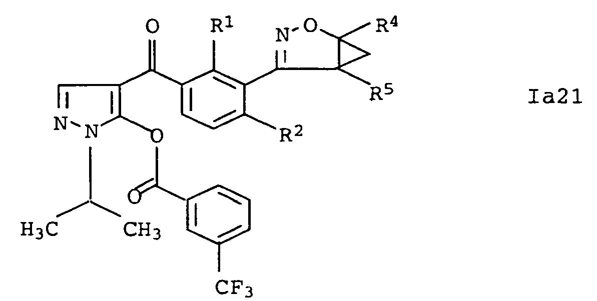

Ebenso außerordentlichst bevorzugt sind die Verbindungen der Formel Ia22, insbesondere die Verbindungen Ia22.1 bis Ia22.77, die sich von den entsprechenden Verbindungen Ia1.1 bis Ia1.77 dadurch unterscheiden, dass R⁶ für 3-Trifluormethylphenylcarbonyloxy und R⁷ für t-Butyl stehen.

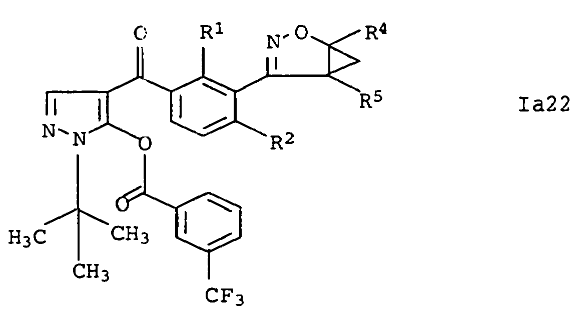

Ebenso auBerordentlichst bevorzugt sind die Verbindungen der Formel Ia23, insbesondere die Verbindungen Ia23.1 bis Ia23.77, die sich von den entsprechenden Verbindungen Ia1.1 bis Ia1.77 dadurch unterscheiden, dass R⁶ für 3-Chlorphenylcarbonyloxy steht.

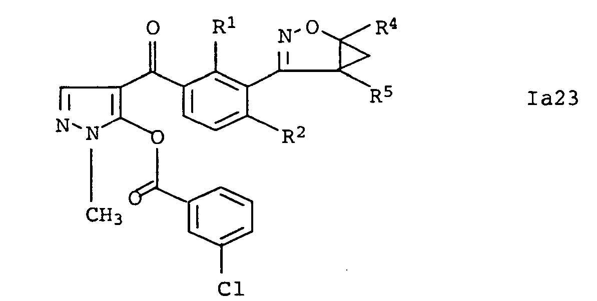

Ebenso außerordentlichst bevorzugt sind die Verbindungen der Formel Ia24, insbesondere die Verbindungen Ia24.1 bis Ia24.77, die sich von den entsprechenden Verbindungen Ia1.1 bis Ia1.77 dadurch unterscheiden, dass R⁶ für 3-Chlorphenylcarbonyloxy und R⁷ für Ethyl stehen.

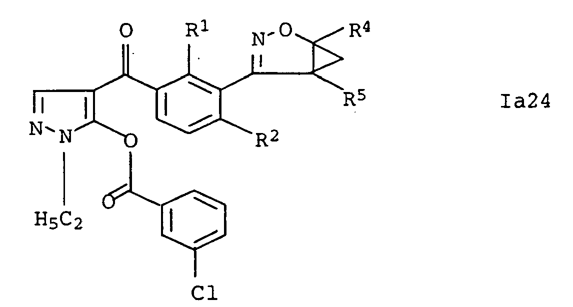

Ebenso außerordentlichst bevorzugt sind die Verbindungen der Formel Ia25, insbesondere die Verbindungen Ia25.1 bis Ia25.77, die sich von den entsprechenden Verbindungen Ia1.1 bis Ia1.77 dadurch unterscheiden, dass R⁶ für 3-Chlorphenylcarbonyloxy und R⁷ für i-Propyl stehen.

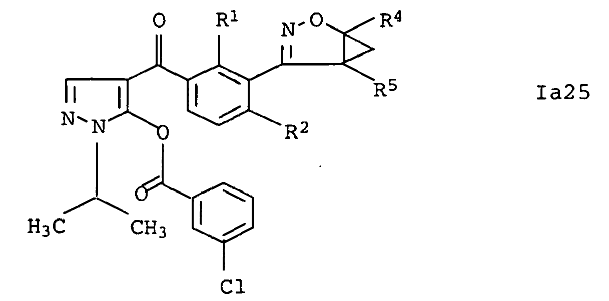

Ebenso außerordentlichst bevorzugt sind die Verbindungen der Formel Ia26, insbesondere die Verbindungen Ia26.1 bis Ia26.77, die sich von den entsprechenden Verbindungen Ia1.1 bis Ia1.77 dadurch unterscheiden, dass R⁶ für 3-Chlorphenylcarbonyloxy und R⁷ für t-Butyl stehen.

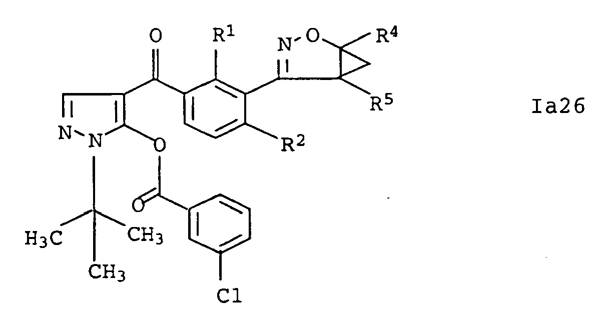

Die Cyclopropyl-anellierten 3-(4,5-Dihydroisoxazol-3-yl)-substituierten Benzoylpyrazole der Formel I sind auf verschiedene Art und Weise erhältlich, beispielsweise nach folgenden Verfahren.

### Verfahren A:

Durch Umsetzung von Pyrazolen der Formel II mit einem aktivierten Benzoesäure-Derivat IIIα oder einer Benzoesäure IIIβ, die vorzugsweise in situ aktiviert wird, zu dem entsprechenden Acylierungsprodukt I' und anschließende Umlagerung erhält man Verbindungen der Formel I mit R⁶ = OH.

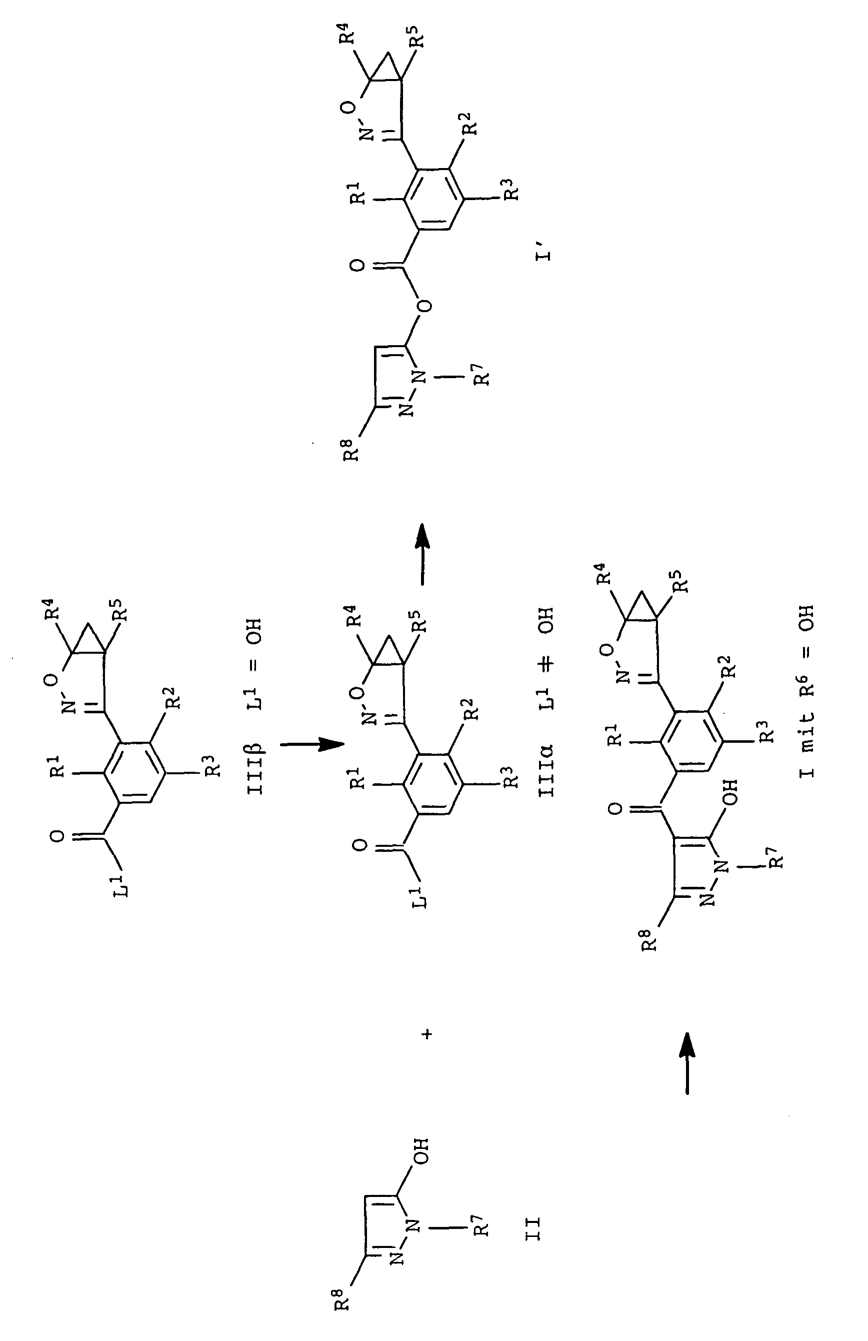

L¹ steht für Hydroxy oder eine nucleophil verdrängbare Abgangsgruppe, wie Halogen z. B. Brom, Chlor, Hetaryl, z. B. Imidazolyl, Pyridyl, Carboxylat, z. B. Acetat, Trifluoracetat, etc.

Das aktivierte Benzoesäure-Derivat kann direkt eingesetzt werden, wie im Fall der Benzoylhalogenide oder in situ erzeugt werden, z. B. mit Dicyclohexylcarbodiimid, Triphenylphosphan/Azodicarbonsäureester, 2-Pyridindisulfid/Triphenylphosphan, Carbonyldiimidazol etc.

Gegebenenfalls kann es von Vorteil sein, die Acylierungsreaktion in Gegenwart einer Base auszuführen. Die Reaktanden und die Hilfsbase werden dabei zweckmäßigerweise in äquimolaren Mengen eingesetzt. Ein geringer Überschuss der Hilfsbase z. B. 1,2 bis 1,5 Moläquivalente, bezogen auf II, kann unter Umständen vorteilhaft sein.

Als Hilfsbasen eignen sich tertiäre Alkylamine, Pyridin oder Alkalimetallcarbonate. Als Lösungsmittel können z. B. chlorierte Kohlenwasserstoffe, wie Methylenchlorid, 1,2-Dichlorethan, aromatische Kohlenwasserstoffe, wie Toluol, Xylol, Chlorbenzol, Ether, wie Diethylether, Methyl-tert.-butylether, Dimethoxyethan, Tetrahydrofuran, Dioxan, polare aprotische Lösungsmittel, wie Acetonitril, Dimethylformamid, Dimethylsulfoxid oder Ester wie Essigsäureethylester oder Gemische hiervon verwendet werden.

Werden Benzoylhalogenide als aktivierte Carbonsäurekomponente eingesetzt, so kann es zweckmäßig sein, bei Zugabe dieses Reaktionspartners die Reaktionsmischung auf 0 - 10 °C abzukühlen. Anschließend rührt man bei 20 - 100 °C, vorzugsweise bei 25 - 50 °C, bis die Umsetzung vollständig ist. Die Aufarbeitung erfolgt in üblicher Weise, z. B. wird das Reaktionsgemisch auf Wasser gegossen, das Wertprodukt extrahiert. Als Lösungsmittel eignen sich hierfür besonders Methylenchlorid, Diethylether, Dimethoxyethan und Essigsäureethylester. Nach Trocknen der organischen Phase und Entfernen des Lösungsmittels kann der rohe Ester I' ohne weitere Reinigung zur Umlagerung eingesetzt werden.

Die Umlagerung der Ester I' zu den Verbindungen der Formel I erfolgt zweckmäßigerweise bei Temperaturen von 20 bis 40 °C in einem Lösungsmittel und in Gegenwart einer Base sowie gegebenenfalls mit Hilfe einer Cyanoverbindung als Katalysator.

Als Lösungsmittel können z. B. Acetonitril, Methylenchlorid, 1,2-Dichlorethan, Dioxan, Essigsäureethylester, Dimethoxyethan, Tetrahydrofuran, Toluol oder Gemische hiervon verwendet werden. Bevorzugte Lösungsmittel sind Acetonitril und Dioxan.

Geeignete Basen sind tertiäre Amine, wie Triethylamin, Pyridin oder Alkalicarbonate, wie Natriumcarbonat, Kaliumcarbonat, die vorzugsweise in äquimolarer Menge oder bis zu einem vierfachen Überschuss, bezogen auf den Ester, eingesetzt werden. Bevorzugt werden Triethylamin oder Alkalicarbonate verwendet, vorzugsweise in doppelt äquimolaren Verhältnis in Bezug auf den Ester.

Als Cyanoverbindungen kommen anorganische Cyanide, wie Natriumcyanid, Kaliumcyanid und organische Cyanoverbindungen, wie Acetoncyanhydrin, Trimethylsilycyanid in Betracht. Sie werden in einer Menge von 1 bis 50 Molprozent, bezogen auf den Ester, eingesetzt. Vorzugsweise werden Acetoncyanhydrin oder Trimethylsilylcyanid, z. B. in einer Menge von 5 bis 15, vorzugsweise 10 Molprozent, bezogen auf den Ester, eingesetzt.

Die Aufarbeitung kann in an sich bekannter Weise erfolgen. Das Reaktionsgemisch wird z. B. mit verdünnter Mineralsäure, wie 5 %ige Salzsäure oder Schwefelsäure, angesäuert, mit einem organischen Lösungsmittel, z. B. Methylenchlorid, Essigsäure-ethylester extrahiert. Der organische Extrakt kann mit 5-10 %iger Alkalicarbonatlösung, z. B. Natriumcarbonat-, Kaliumcarbonatlösung extrahiert werden. Die wässrige Phase wird angesäuert und der sich bildende Niederschlag abgesaugt und/oder mit Methylenchlorid oder Essigsäureethylester extrahiert, getrocknet und eingeengt. (Beispiele für die Darstellung von Estern von Hydroxypyrazolen und für die Umlagerung der Ester sind z. B. in EP-A 282 944 und US 4 643 757 genannt).

Es ist aber auch möglich, den Ester I' in situ zu erzeugen, indem man ein Pyrazol der Formel II, oder ein Alkalisalz hiervon, mit einem Cyclopropyl-anellierten 3-(4,5-Dihydroisoxazol-3-yl)-benzol-Derivat der Formel IV in Gegenwart von Kohlenmonoxid, eines Katalysators sowie einer Base, umsetzt.

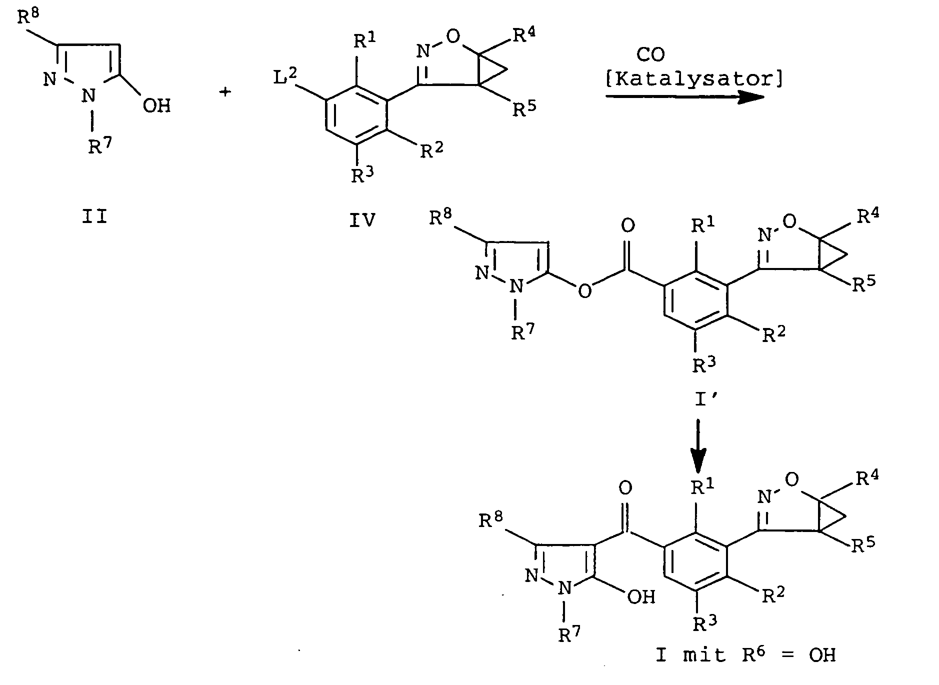

L² steht für eine Abgangsgruppe wie Halogen, z. B. Chlor, Brom oder Iod, oder Sulfonat wie Mesylat oder Triflat; bevorzugt sind Brom oder Triflat.

Der Ester I' reagiert ggf. direkt zu dem Cyclopropyl-anellierten 3-(4,5-Dihydroisoxazol-3-yl)-substituierten Benzoylpyrazol der Formel I ab.

Als Katalysatoren eignen sich Palladiumligandkomplexe, in denen das Palladium in der Oxidationsstufe 0 vorliegt, metallisches Palladium, das gegebenenfalls auf einen Träger aufgezogen wurde, und vorzugsweise Palladium(II)salze. Die Umsetzung mit Palladium(II)salzen und metallischem Palladium wird vorzugsweise in Gegenwart von Komplexliganden durchgeführt.

Als Palladium (0) ligandkomplex kommt beispielsweise Tetrakis(triphenylphosphan)palladium in Frage.

Metallisches Palladium ist vorzugsweise auf einen inerten Träger wie beispielsweise Aktivkohle, Siliciumdioxid, Aluminiumoxid, Bariumsulfat oder Calciumcarbonat aufgezogen. Die Reaktion wird vorzugsweise in Gegenwart von Komplexliganden wie beispielsweise Triphenylphosphan durchgeführt.

Als Palladium(II)salze eigenen sich beispielsweise Palladiumacetat und Palladiumchlorid. Bevorzugt wird in Gegenwart von Komplexliganden wie beispielsweise Triphenylphosphan gearbeitet.

Geeignete Komplexliganden für die Palladiumligandkomplexe, bzw. in deren Gegenwart die Umsetzung mit metallischem Palladium oder Palladium(II)salzen vorzugsweise ausgeführt wird, sind tertiäre Phosphane, deren Struktur durch folgende Formeln wiedergegeben wird: 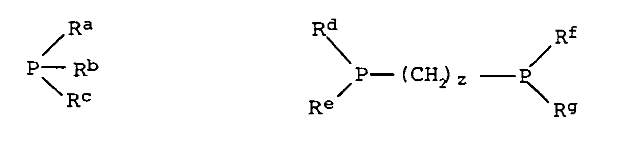 wobei z die Zahlen 1 bis 4 bedeutet und die Reste R^{a} bis R^{g} für C₁-C₆-Alkyl, C₃-C₆-Cycloalkyl, Aryl-C₁-C₂-alkyl oder vorzugsweise Aryl stehen. Aryl steht beispielsweise für Naphthyl und gegebenenfalls substituiertes Phenyl wie beispielsweise 2-Tolyl und insbesondere für unsubstituiertes Phenyl.

Die Herstellung der komplexen Palladiumsalze kann in an sich bekannter Weise ausgehend von kommerziell erhältlichen Palladiumsalzen wie Palladiumchlorid oder Palladiumacetat und den entsprechenden Phosphanen wie z. B. Triphenylphosphan, Tricyclohexylphosphan oder 1,2-Bis(diphenylphosphano)ethan erfolgen. Ein Großteil der komplexierten Palladiumsalze ist auch kommerziell erhältlich. Bevorzugte Palladiumsalze sind [(R)(+)2,2'-Bis(diphenylphosphano)-1,1'-binaphthyl]palladium(II)chlorid, Bis(triphenylphosphan)palladium(II)acetat und insbesondere Bis(triphenylphosphan)palladium (II) chlorid.

Der Palladiumkatalysator wird in der Regel in einer Konzentration von 0,05 bis 5 Mol-%, bevorzugt 1-3 Mol-%, eingesetzt.

Als Basen kommen tertiäre Amine wie beispielsweise N-Methylpiperidin, Ethyldiisopropylamin, 1,8-Bisdimethylaminonaphthalin oder insbesondere Triethylamin in Betracht. Ebenso eigenen sich Alkalicarbonat, wie Natriumcarbonat oder Kaliumcarbonat. Aber auch Gemische von Kaliumcarbonat und Triethylamin sind geeignet.

In der Regel werden 2 bis 4 Moläquivalente, insbesondere 2 Moläquivalente, des Alkalicarbonats, sowie 1 bis 4 Moläquivalente, insbesondere 2 Moläquivalente des tertiären Amins bezogen auf das Cyclopropyl-anellierte 3-(4,5-Dihydroisoxazol-3-yl)-benzol-Derivat der Formel IV eingesetzt.

Als Lösungsmittel können Nitrile, wie Benzonitril und Acetonitril, aromatische Kohlenwasserstoffe, wie Toluol, Amide, wie Dimethylformamid, Dimethylacetamid, Tetra-C₁-C₄-alkylharnstoffe oder N-Methylpyrrolidon und vorzugsweise Ether wie Tetrahydrofuran, Methyl-tert.-butylether dienen. Insbesondere werden Ether, wie 1,4-Dioxan und Dimethoxyethan als Lösungsmittel bevorzugt.

Die Verbindungen der Formel IIIß sind z. B. wie folgt erhältlich:

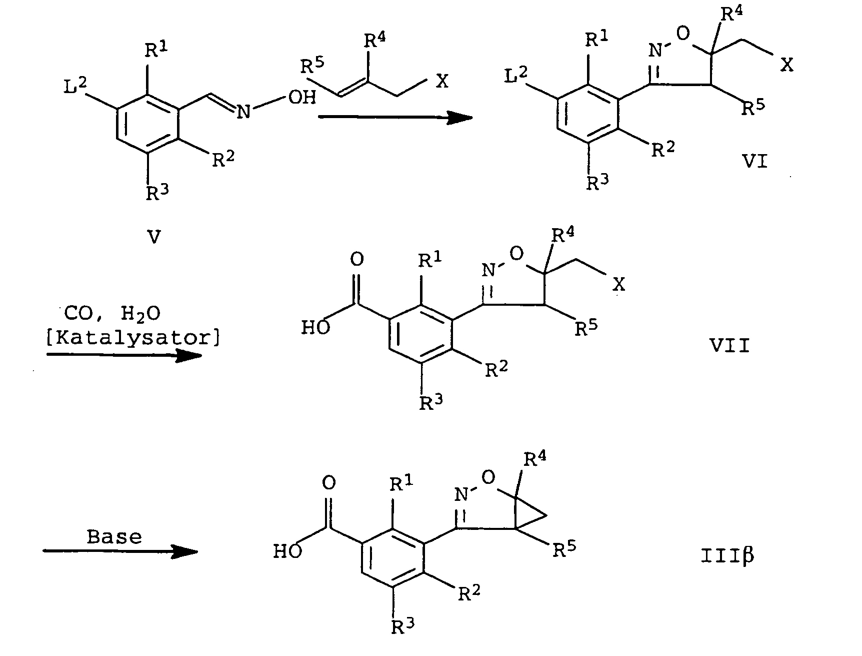

Die Umwandlung von Oximen der Formel V in die 4,5-Dihydroisoxazol-3-yl-benzolderivate VI kann in an sich bekannter Weise über die Zwischenstufe der Hydroxamsäurehalogenide, insbesondere über die Hydroxamsäurechloride, erfolgen. Aus diesen werden in situ Nitriloxide erzeugt, die mit Alkenen zu den gewünschten Produkten abreagieren (vgl. z. B. Chem. Ber. 106, 3258-3274 (1973)). So wird das Oxim V z. B. mit Natriumhypochlorit oxidiert und mit einem Allylhalogenid, z. B. Allylchlorid, in einem inerten Lösungsmittel, wie Methylenchlorid, Chloroform, Tetrahydrofuran, Dioxan oder Acetonitril zum (4,5-Dihydroisoxazol-3-yl)benzol-Derivat VI umgesetzt. Dieses wird dann in Gegenwart eines Katalysators sowie einer Base mit Kohlenmonoxid und Wasser zu VII umgesetzt.

L² steht für eine Abgangsgruppe wie Halogen, z. B. Chlor, Brom oder Iod, oder Sulfonat wie Mesylat oder Triflat; bevorzugt sind Brom oder Triflat.

X steht für Halogen, vorzugsweise Chlor oder Brom.

Als Katalysatorsysteme eignen sich die voranstehend beschriebenen Palladiumligandkomplexe. Die Reaktionsbedingungen gelten in Analogie.

Der Ringschluss, also die Umsetzung der Veribndung VII zu der Verbindung IIIß, zum Cyclopropanring erfolgt unter Anwendung starker Basen, wie Alkalimetallalkoxiden, z. B. Kalium-tert-butylat, vorzugsweise in polaren aprotischen Lösungsmitteln, wie Dimethylsulfoxid.

Der Ringschluss zum Cyclopropanring kann auch auf der Stufe der Verbindung VI erfolgen, wobei die Verbindung IV erhalten wird, die in Analogie mit Kohlenmonoxid und Wasser in Gegenwart eines Katalysators und Base zu IIIß weiter umgesetzt werden kann.

Ebenso ist es möglich, die Verbindungen der Formel IIIß zu erhalten, indem man ein Oxim der Formel VIII in das entsprechende Hydroxamsäurehalogenid, insbesondere Hydroxamsäurechlorid, überführt, in situ ein Nitriloxid erzeugt und dieses mit einem Alken umsetzt (vlg. z. B. Chem. Ber. 106, 3258-3274 (1973)). Anschlie-ßend wird der Ester unter an sich bekannten Bedingungen verseift zu dem (4,5-Dihydroisoxazol-3-yl)-benzol VII und wie voranstehend beschrieben weiter umgesetzt.

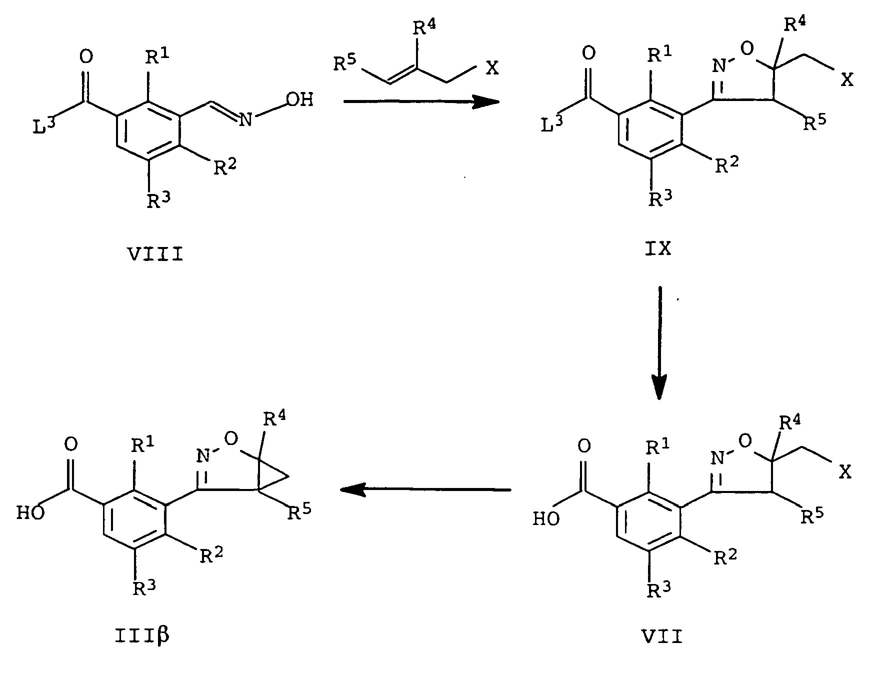

L³ steht für einen C₁-C₆-Alkoxy-Rest und X für Halogen, vorzugsweise Chlor oder Brom.

Verbindungen der Formel I mit R⁶ ≠ Hydroxy werden durch Umsetzung von Verbindungen der Formel I mit R⁶ = Hydroxy mit Alkylierungsmitteln, Sulfonylierungsmittel bzw. Acylierungsmitteln L⁴-R^{6a} (X) erhalten.

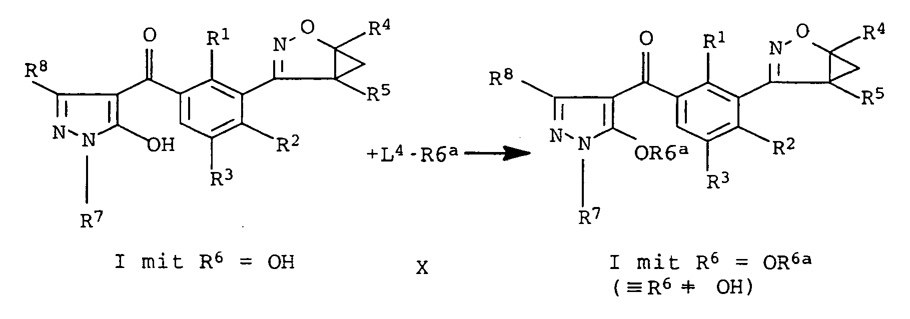

L⁴ steht für eine nucleophil verdrängbare Abgangsgruppe, wie Halogen, z. B. Brom oder Chlor, Acyloxy, z. B. Acetyloxy, Ethylcarbonyloxy, oder Alkylsulfonyloxy, z. B. Methylsulfonyloxy oder Trifluormethylsulfonyloxy;

R^{6a} steht für C₁-C₆-Alkyl, C₃-C₆-Alkenyl, C₁-C₆-Alkylsulfonyl, C₁-C₆-Alkylcarbonyl, Phenyl-C₁-C₄-alkyl, Phenylcarbonyl-C₁-C₄-alkyl, Phenylsulfonyl oder Phenylcarbonyl, wobei der Phenylrest der vier letztgenannten Substituenten partiell oder vollständig halogeniert sein kann und/oder eine bis drei der folgenden Gruppen tragen kann: Nitro, Cyano, C₁-C₄-Alkyl, C₁-C₄-Halogenalkyl, C₁-C₄-Alkoxy oder C₁-C₄-Halogenalkoxy.

Die Verbindungen der Formel X können direkt eingesetzt werden, wie z. B. im Fall der Sulfonsäurehalogenide, Sulfonsäureanhydride oder in situ erzeugt werden, z. B. aktivierte Sulfonsäuren (mittels Sulfonsäure und Dicyclohexylcarbonyldiimid, Carbonyldiimidazol etc.).

Die Ausgangsverbindungen werden in der Regel im äquimolaren Verhältnis eingesetzt. Es kann aber auch von Vorteil sein, die eine oder andere Komponente im Überschuss einzusetzen.

Gegebenenfalls kann es von Vorteil sein, die Umsetzung in Gegenwart einer Base durchzuführen. Die Reaktanden und die Hilfsbase werden dabei zweckmäßigerweise in äquimolaren Mengen eingesetzt. Ein Überschuss der Hilfsbase, z. B. 1,5 bis 3 Moläquivalente, bezogen auf I (mit R⁶ = OH), kann unter Umständen vorteilhaft sein.

Als Hilfsbasen eignen sich tertiäre Alkylamine, wie Triethylamin, Pyridin, Alkalimetallcarbonate, z. B. Natriumcarbonat, Kaliumcarbonat und Alkalimetallhydride, z. B. Natriumhydrid. Bevorzugt verwendet werden Triethylamin und Pyridin.

Als Lösungsmittel kommen z. B. chlorierte Kohlenwasserstoffe, wie Methylenchlorid, 1,2-Dichlorethan, aromatische Kohlenwasserstoffe, z. B. Toluol, Xylol, Chlorbenzol, Ether, wie Diethylether, Methyl-tert.-butylether, Tetrahydrofuran, Dioxan, polare aprotische Lösungsmittel, wie Acetonitril, Dimethylformamid, Dimethylsulfoxid oder Ester, wie Essigsäureethylester, oder Gemische hiervon in Betracht.

In der Regel liegt die Reaktionstemperatur im Bereich von 0 °C bis zur Höhe des Siedepunktes des Reaktionsgemisches.

Die Aufarbeitung kann in an sich bekannter Weise zum Produkt hin erfolgen.

Die Pyrazole der Formel II sind bekannt oder können nach an sich bekannten Verfahren hergestellt werden (z. B. EP-A 240 001 und J. Prakt. Chem. 315, 383 (1973)).

Die Verbindungen der Formel IIIa und IV sind als solche jeweils neu, 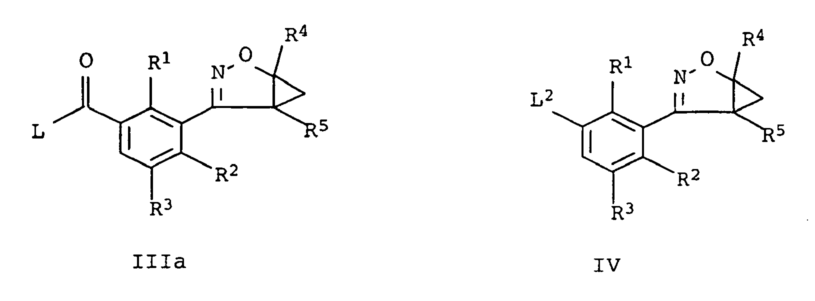 wobei jeweils die Variablen R¹ bis R⁵ die bei den Verbindungen der Formel I gegebene Bedeutung haben und
L für Hydroxy oder einen abhydrolysierbaren Rest steht;
bzw.

L² für eine nukleophil verdrängbare Abgangsgruppe steht.

Beispiele für abhydrolysierbare Reste sind Alkoxy-, Phenoxy-, Alkylthio-, Phenylthio-Reste, die gegebenenfalls substituiert sein können, Halogenide, Hetarylreste, die über Stickstoff gebunden sind, Amino-, Imino-Reste, die gegebenenfalls substituiert sein können etc.

Beispiele für eine nukleophil verdrängbare Abgangsgruppe sind Halogen, C₁-C₄-Alkylsulfonyloxy oder C₁-C₄-Halogenalkylsulfonyloxy.

Bevorzugte Verbindungen der Formel III sind diejenigen, wobei L für Halogen, insbesondere Chlor oder Brom steht.

Ebenso bevorzugt sind diejenigen Verbindungen der Formel III, wobei L Hydroxy bedeutet.

Ebneso bevorzugt sind diejenigen Verbindungen der Formel III, wobei L für C₁-C₆-Alkoxy steht.

Die besonders bevorzugten Ausführungsformen der Verbindungen der Formel III bzw. IV im Hinblick auf die Variablen R¹ bis R⁵ entsprechen denen der Cyclopropyl-anellierten 3-(4,5-Dihydroisoxazol-3-yl)-substituierten Benzolpyrazole der Formel I.

### Verfahren B:

Alternativ können die Verbindungen der Formel I mit R⁶ = OH wie folgt hergestellt werden:

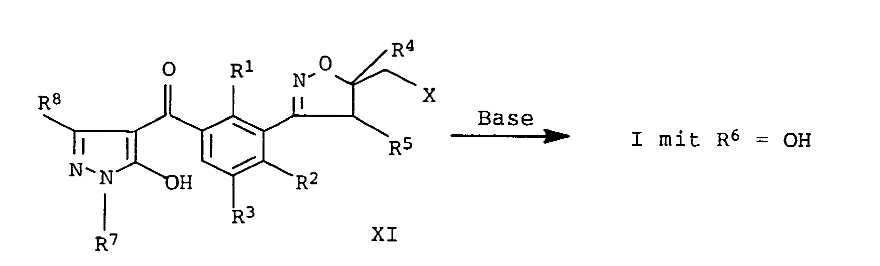

Geeignete Basen und Lösungsmittel sind die oben für dem Ringschluss genannten.

Die Verbindungen der Formel I mit R⁶ = OH können, wie oben erörtert, durch Umsetzung mit Alkylierungsmitteln, Sulfonylierungsmitteln bzw. Acylierungsmitteln L⁴-R^{6a} (X) in Verbindungen der Formel I mit R⁶ ≠ OH umgewandelt werden.

### Herstellungsbeispiele

### 4-[2-Methyl-3-(2-oxa-3-aza-bicyclo[3.1.0]hex-3-en-4-yl)-4-methylsulfonylbenzoyl]-5-hydroxy-1-methyl-1H-pyrazol (Verbindung 2.3)

### Stufe a)

Zu einer Lösung von 3,0 g (9 mmol) 2-Methyl-3-(5-chlormethyl-4,5-dihydroisoxazol-3-yl)-4-methylsulfonylbenzoesäure in 5 ml Dimethylsulfoxid wurden bei 15-20 °C 3,04 g Kalium-t-butylat gegeben und über Nacht bei Raumtemperatur gerührt. Das Reaktionsgemisch wurde in 0,3 1 3 %ige Salzsäure eingerührt und dreimal mit Essigsäure-ethylester extrahiert. Man wusch die vereinigten organischen Phasen mit Wasser, trocknete und entfernte das Lösungsmittel. Der Rückstand wurde säulenchromatographisch an Kieselgel gereinigt (Elutionsmittel = Toluol/Tetrahydrofuran/Essigsäure 8/2/1). Man erhielt 1,3 g (49 % d. Th.) 2-Methyl-3-(2-oxa-3-aza-bicyclo[3.1.0]hex-3-en-4-yl)-4-methylsulfonylbenzoesäure. Das gefundene ¹H-NMR-Spektrum entsprach der angegebenen Struktur.

### Stufe b)

Zu einer Lösung von 0,75 g (2,54 mmol) des Produktes aus Stufe a) und 0,25 g (2,54 mmol) 1-Methyl-5-hydroxy-1H-pyrazol in 50 ml wasserfreiem Acetonitril gab man unter einer Stickstoffatmosphäre 0,79 g (3,9 mmol) Dicyclohexylcarbodiimid und rührte 2 Stunden bei 40 °C und weitere 12 Stunden bei Raumtemperatur. Das Lösungsmittel wurde unter Abdampfen unter vermindertem Druck entfernt; der Rückstand wurde in Essigsäureethylester aufgenommen, dreimal mit 5 %igem Kaliumcarbonat und dreimal mit Wasser gewaschen, getrocknet und zur Trockene eingeengt. Man erhielt 1,2 g eines zähen braunen Öls, das ohne Reinigung für die weitere Umsetzung verwendet wurde.

### Stufe c)

Ein Gemisch aus 1,2 g des Produktes aus Stufe b) und 0,52 g Kaliumcarbonat in 5 ml Dioxan wurde 4 Stunden bei Raumtemperatur gerührt. Anschließend engte man das Gemisch unter vermindertem Druck zur Trockene ein, nahm den Rückstand in Wasser auf und wusch die wässrige Phase dreimal mit Diethylether. Der pH-Wert der wässrigen Phase wurde dann mit 10 %iger Salzsäure auf 1-2 eingestellt; die wässrige Phase wurde dreimal mit Essigsäureethylester extrahiert. Die vereinigten organischen Phasen wurden mit Wasser gewaschen, getrocknet und unter vermindertem Druck zur Trockene eingeengt. Man erhielt 0,3 g (33 % d. Th.) eines amorphen Schaums. Das gefundene ¹H-NMR-Spektrum entsprach der angegebenen Struktur der Titelverbindung.

### 4-(2-Methyl-3-(2-oxa-3-aza-bicyclo[3.1.0]hex-3-en-4-yl)-4-methylsulfonylbenzoyl]-5-hydroxy-1-t-butyl-1H-pyrazol (Verbindung 2.1)

Zu einer Lösung von 0,23 g (0,51 mmol) 4-[2-Methyl-3-(5-chlormethyl-4,5-dihydro-isoxazol-3-yl)-4-methylsulfonylbenzoyl]-5-hydroxy-1-t-butyl-1H-pyrazol in 2,5 ml Dimethylsulfoxid gab man bei Raumtemperatur 0,17 g (1,50 mmol) Kalium-tert-butylat und rührte über Nacht. Anschließend rührte man die Mischung in 300 ml 3 %ige wässrige Salzsäure ein und extrahierte die wässrige Phase dreimal mit je 200 ml Essigsäureethylester, wusch die vereinigten organischen Phasen viermal mit je 50 ml Wasser, trocknete über Natriumsulfat und engte im Vakuum ein. Man erhielt 0,12 g (57%) der Titelverbindung in Form eines braunen Feststoffs mit einem Schmelzpunkt von 69-74 °C.
¹H-NMR (δ in ppm): δ = 1, 1-1, 3 (m, 2H), 1,58 (s, 9H), 2,58 (s, 3H), 2,3-2,5 (m, 1H), 3,22 (s, 3H), 5,21 (m, 1H), 7,32 (s, 1H), 7,72 (d, 1H), 8,11 (d, 1H) .

In Tabelle 2 sind neben der voranstehenden Verbindung noch weitere Cyclopropyl-anellierte 3-(4,5-Dihydroisoxazol-3-yl)-substituierte Benzoylpyrazole der Formel I aufgeführt, die in analoger Weise hergestellt wurden oder herstellbar sind.

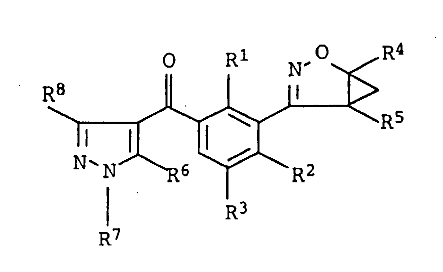

**Tabelle 2:**

| Nr. | R¹ | R² | R³ | R⁴ | R⁵ | R⁶ | R⁷ | R⁸ | physikalische Daten Fp[°C] |
|---|---|---|---|---|---|---|---|---|---|
| 2.1 | CH₃ | SO₂CH₃ | H | H | H | OH | C(CH₃)₃ | H | 69-74 |
| 2.2 | CH₃ | SO₂CH₃ | H | H | H | OH | C₂H₅ | H | 94-97 |
| 2.3 | CH₃ | SO₂CH₃ | H | H | H | OH | CH₃ | H | 106-112 |
| 2.4 | CH₃ | SO₂CH₃ | H | H | H | OH | CH(CH₃)₂ | H | 90-95 |
| 2.5 | CH₃ | SO₂CH₃ | H | H | H | OH | CH₃ | CH₃ | 89-91 |
| 2.6 | CH₃ | SO₂CH₃ | H | CH₂Cl | H | OH | CH₂CH₃ | H | 99-103 |
| 2.7 | CH₃ | SO₂CH₃ | H | CH₃ | H | OH | CH₃ | H | 98-102 |
| 2.8 | CH₃ | SO₂CH₃ | H | -CH₂- | | OH | CH(CH₃)₂ | H | 115-119 |
| 2.9 | CH₃ | SO₂CH₃ | H | -CH₂- | | OH | C(CH₃)₃ | H | 136-140 |
| 2.10 | CH₃ | SO₂CH₃ | H | CH₃ | H | OH | C(CH₃)₃ | H | 90-95 |
| 2.11 | CH₃ | SO₂CH₃ | H | CH₃ | H | OH | CH(CH₃)₂ | H | 67-70 |
| 2.12 | CH₃ | SO₂CH₃ | H | CH₃ | H | OH | cyclo-C₃H₅ | H | 71-79 |
| 2.13 | CH₃ | SO₂CH₃ | H | H | CH₃ | OH | CH₃ | H | 99-103 |
| 2.14 | CH₃ | SO₂CH₃ | H | H | CH₃ | OH | CH(CH₃)₂ | H | 75-80 |
| 2.15 | CH₃ | SO₂CH₃ | H | H | CH₃ | OH | cyclo-C₃H₅ | H | 65-70 |
| 2.16 | CH₃ | SO₂CH₃ | H | H | CH₃ | OH | C(CH₃)₃ | H | 110-115 |

Die Cyclopropyl-anellierten 3-(4,5-Dihydroisoxazol-3-yl)-substituierten Benzoylpyrazole der Formel I und deren landwirtschaftlich brauchbaren Salze eignen sich - sowohl als Isomerengemische als auch in Form der reinen Isomeren - als Herbizide. Die Verbindungen der Formel I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen der Formel I bzw. sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa , Phaseolus lunatus, Phaseolus vulgaris, Picea abies, Pinus spec., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Darüber hinaus können die Verbindungen der Formel I auch in Kulturen, die durch Züchtung einschließlich gentechnischer Methoden gegen die Wirkung von Herbiziden tolerant sind, verwandt werden.

Die Verbindungen der Formel I bzw. die sie enthaltenden herbiziden Mittel können beispielsweise in Form von direkt versprühbaren wässrigen Lösungen, Pulvern, Suspensionen, auch hochprozentigen wässrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die herbiziden Mittel enthalten eine herbizid wirksame Menge mindestens einer Verbindung der Formel I oder eines landwirtschaftlich brauchbaren Salzes von I und für die Formulierung von Pflanzenschutzmitteln übliche Hilfsmittel.

Als inerte Hilfsstoffe kommen im Wesentlichen in Betracht: Mineralölfraktionen von mittlerem bis hohem Siedepunkt, wie Kerosin und Dieselöl, ferner Kohlenteeröle sowie Öle pflanzlichen oder tierischen Ursprungs, aliphatische, cyclische und aromatische Kohlenwasserstoffe, z. B. Paraffine, Tetrahydronaphthalin, alkylierte Naphthaline und deren Derivate, alkylierte Benzole und deren Derivate, Alkohole wie Methanol, Ethanol, Propanol, Butanol und Cyclohexanol, Ketone wie Cyclohexanon, stark polare Lösungsmittel, z. B. Amine, wie N-Methylpyrrolidon und Wasser.

Wässrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Cyclopropyl-anellierten 3-(4,5-Dihydroisoxazol-3-yl)-substituierten Benzoylpyrazole als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe (Adjuvantien) kommen die Alkali-, Erdalkali-, Ammoniumsalze von aromatischen Sulfonsäuren, z. B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate können durch Bindung der Wirkstoffe an feste Trägerstoffe hergestellt werden. Feste Trägerstoffe sind Mineralerden, wie Kieselsäuren, Kieselgele, Silikate, Talkum, Kaolin, Kalkstein, Kalk, Kreide, Bolus, Löss, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nussschalenmehl, Cellulosepulver oder andere feste Trägerstoffe.

Die Konzentrationen der Verbindungen der Formel I in den anwendungsfertigen Zubereitungen können in weiten Bereichen variiert werden. Im Allgemeinen enthalten die Formulierungen etwa von 0,001 bis 98 Gew.-%, vorzugsweise 0,01 bis 95 Gew.-%, mindestens eines Wirkstoffs. Die Wirkstoffe werden dabei in einer Reinheit von 90 % bis 100 %, vorzugsweise 95 % bis 100 % (nach NMR-Spektrum) eingesetzt.

Die folgenden Formulierungsbeispiele verdeutlichen die Herstellung solcher Zubereitungen:
I. 20 Gewichtsteile der Verbindung Nr. 2.5 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Rizinusöl besteht. Durch Ausgießen und feines Verteilen der Lösung in 100 000 Gewichtsteilen Wasser erhält man eine wässrige Dispersion, die 0,02 Gew.-% des Wirkstoffs enthält.
II. 20 Gewichtsteile der Verbindung Nr. 2.5 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Rizinusöl besteht. Durch Eingießen und feines Verteilen der Lösung in 100 000 Gewichtsteilen Wasser erhält man eine wässrige Dispersion, die 0,02 Gew.-% des Wirkstoffs enthält.
III. 20 Gewichtsteile des Wirkstoffs Nr. 2.5 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wässrige Dispersion, die 0,02 Gew.-% des Wirkstoffs enthält.
IV. 20 Gewichtsteile des Wirkstoffs Nr. 2.5 werden mit 3 Gewichtsteilen des Natriumsalzes der Diisobutylnaphthalinsulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.-% des Wirkstoffs enthält.
V. 3 Gewichtsteile des Wirkstoffs Nr. 2.5 werden mit 97 Gewichtsteilen feinteiligem Kaolin vermischt. Man erhält auf diese Weise ein Stäubemittel, das 3 Gew.-% des Wirkstoffs enthält.
VI. 20 Gewichtsteile des Wirkstoffs Nr. 2.5 werden mit 2 Gewichtsteilen Calciumsalz der Dodecylbenzolsulfonsäure, 8 Gewichtsteilen Fettalkoholpolyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
VII. 1 Gewichtsteil des Wirkstoffs Nr. 2.5 wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
VIII. 1 Gewichtsteil des Wirkstoffs Nr. 2.5 wird in einer Mischung gelöst, die aus 80 Gewichtsteilen Cyclohexanon und 20 Gewichtsteilen Wettol^{®} EM 31 (= nichtionischer Emulgator auf der Basis von ethoxyliertem Rizinusöl) besteht. Man erhält ein stabiles Emulsionskonzentrat.

Die Applikation der Verbindungen der Formel I bzw. der herbiziden Mittel kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, dass die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Verbindung der Formel I betragen je nach Bekämpfungsziel, Jahreszeit, Zielpflanzen und Wachstumsstadium 0,001 bis 3,0, vorzugsweise 0,01 bis 1,0 kg/ha aktive Substanz (a.S.) .

Zur Verbreiterung des Wirkungsspektrums und zur Erzielung synergistischer Effekte können die Cyclopropyl-anellierten 3-(4,5-Dihydroisoxazol-3-yl)-substituierten Benzoylpyrazole der Formel I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyalkansäuren und deren Derivate, Benzoesäure und deren Derivate, Benzothiadiazinone, 2-(Hetaroyl/Aroyl)-1,3-cyclohexandione, Heteroaryl-Aryl-Ketone, Benzylisoxazolidinone, meta-CF₃-Phenylderivate, Carbamate, Chinolincarbonsäure und deren Derivate, Chloracetanilide, Cyclohexenonoximetherderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- und Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide und Uracile in Betracht.

Außerdem kann es von Nutzen sein, die Verbindungen der Formel I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Anwendungsbeispiele

Die herbizide Wirkung der Cyclopropyl-anellierten 3-(4,5-Dihydroisoxazol-3-yl)-substituierten Benzoylpyrazole der Formel I ließ sich durch die folgenden Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 0,25 bzw. 0,125 kg/ha a.S. (aktive Substanz).

Die Pflanzen wurden artenspezifisch bei Temperaturen von 10 bis 25 °C bzw. 20 bis 35 °C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Amaranthus retroflexus | Fuchsschwanz | pig weed |
| Avena fatua | Flughafer | wild oat |
| Chenopodium album | Weißer Gänsefuß | lambsquaters |
| Echinochloa crus galli | Hühnerhirse | barnyardgrass |
| Polygonum persicaria | Flohknöterich | ladysthumb |
| Setaria faberi | Große Borstenhirse | giant foxtail |

Bei Aufwandungen von 0,25 bzw. 0,125 kg/ha zeigte die Verbindung Nr. 2.5 (Tabelle 2) im Nachauflauf eine sehr gute Wirkung gegen oben genannte unerwünschte Planzen.

## Patentansprüche

1. Cyclopropyl-anellierte 3-(4,5-Dihydroisoxazol-3-yl)-substituierte Benzoylpyrazole der Formel I 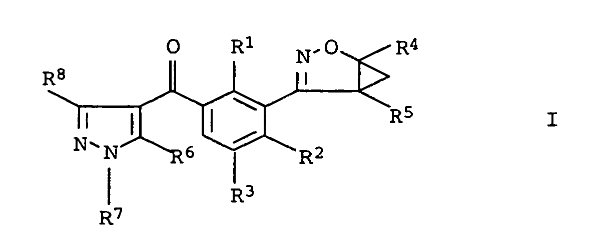 worin
R¹ für C₁-C₆-Alkyl, C₁-C₆-Halogenalkyl, C₁-C₆-Alkoxy, C₁-C₆-Halogenalkoxy, Halogen oder Nitro;
R² für C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, Halogen, Cyano oder Nitro;
R³ für Wasserstoff, C₁-C₆-Alkyl oder Halogen;
R⁴ für Wasserstoff, C₁-C₄-Alkyl oder C₁-C₄-Halogenalkyl steht;
R⁵ die für R⁴ angegebenen Bedeutungen einnehmen kann; oder
R⁴, R⁵ gemeinsam für eine C₁-C₄-Alkandiylgruppe stehen, die partiell oder vollständig halogeniert sein kann und/oder eine bis drei C₁-C₄-Alkylgruppen tragen kann;
R⁶ für Hydroxy, C₁-C₆-Alkoxy, C₃-C₆-Alkenyloxy, C₁-C₆-Alkylsulfonyloxy, C₁-C₆-Alkylcarbonyloxy, Phenyl-C₁-C₄-alkoxy, Phenylcarbonyl-C₁-C₄-alkoxy, Phenylsulfonyloxy, Phenylcarbonyloxy, wobei der Phenylrest der vier letztgenannten Substituenten partiell oder vollständig halogeniert sein kann und/oder eine bis drei der folgenden Gruppen tragen kann: Nitro, Cyano, C₁-C₄-Alkyl, C₁-C₄-Halogenalkyl, C₁-C₄-Alkoxy oder C₁-C₄-Halogenalkoxy;
R⁷ für Wasserstoff, C₁-C₆-Alkyl oder Cyclopropyl;
R⁸ für Wasserstoff, C₁-C₆-Alkyl oder C₁-C₆-Halogenalkyl steht;
sowie deren landwirtschaftlich brauchbaren Salze.

2. Cyclopropyl-anellierte 3-(4,5-Dihydroisoxazol-3-yl)-substituierte Benzoylpyrazole nach Anspruch 1, worin
R¹ für C₁-C₆-Alkyl oder Halogen;
R² für C₁-C₆-Halogenalkyl, C₁-C₆-Alkylsulfonyl oder Halogen;
R³ für Wasserstoff, C₁-C₄-Alkyl oder Halogen;
R⁷ für C₁-C₄-Alkyl oder Cyclopropyl;
R⁸ für Wasserstoff oder C₁-C₄-Alkyl steht.

3. Cyclopropyl-anellierte 3-(4,5-Dihydroisoxazol-3-yl)-substituierte Benzoylpyrazole nach Anspruch 1 oder 2, worin _{R}⁶ für Hydroxy, Phenyl-C₁-C₂-alkoxy, Phenylcarbonyl-C₁-C₂-alkoxy, Phenylsulfonyloxy oder Phenylcarbonyloxy steht, wobei der Phenylrest der vier letztgenannten Substituenten partiell oder vollständig halogeniert sein kann und/oder eine bis drei der folgenden Gruppen tragen kann: Nitro, Cyano, C₁-C₄-Alkyl, C₁-C₄-Halogenalkyl, C₁-C₄-Alkoxy oder C₁-C₄-Halogenalkoxy,

4. Verfahren zur Herstellung von Cyclopropyl-anellierten 3-(4,5-Dihydroisoxazol-3-yl)-substituierten Benzoylpyrazolen nach Anspruch 1, bei dem man ein Pyrazol der Formel II 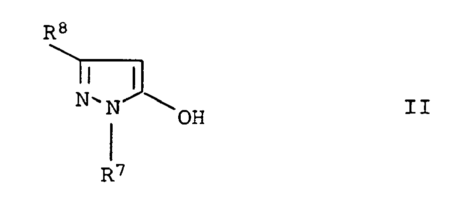 mit einem Benzoesäurederivat der Formel III, 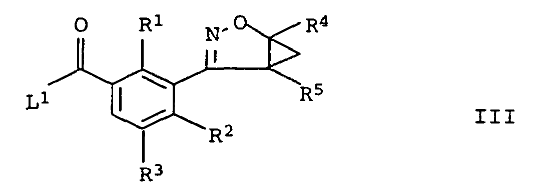 worin die Variablen R¹ bis R⁵, R⁷ und R⁸ die in Anspruch 1 angegebene Bedeutung haben und L¹ für Hydroxy oder eine nucleophil verdrängbare Abgangsgruppe steht, acyliert;
das Acylierungsprodukt zu einer Verbindung der Formel I, worin R⁶ für Hydroxy steht, umlagert und das Umlagerungsprodukt gegebenenfalls mit einer Verbindung der Formel X
L⁴-R^{6a} X
umsetzt, worin
L⁴ für eine nucleophil verdrängbare Abgangsgruppe; und
R^{6a} für C₁-C₆-Alkyl, C₃-C₆-Alkenyl, C₁-C₆-Alkylsulfonyl, C₁-C₆-Alkylcarbonyl, Phenyl-C₁-C₄-alkyl, Phenylcarbonyl-C₁-C₄-alkyl, Phenylsulfonyl oder Phenylcarbonyl, wobei der Phenylrest der vier letztgenannten Substituenten partiell oder vollständig halogeniert sein kann und/oder eine bis drei der folgenden Gruppen tragen kann: Nitro, Cyano, C₁-C₄-Alkyl, C₁-C₄-Halogenalkyl, C₁-C₄-Alkoxy oder C₁-C₄-Halogenalkoxy;
steht.

5. Verfahren zur Herstellung von Cyclopropyl-anellierten 3-(4,5-Dihydroisoxazol-3-yl)-substituierten Benzoylpyrazolen nach Anspruch 1, bei dem man ein Pyrazol der Formel II 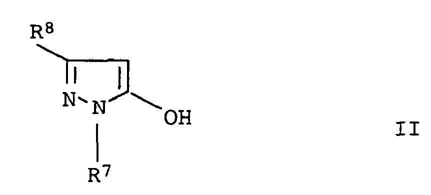 in der die Variablen R⁷ und R⁸ die in Anspruch 1 genannte Bedeutung haben, oder ein Alkalisalz hiervon, mit einem Cyclopropyl-anellierten 3-(4,5-Dihydroisoxazol-3-yl)-benzolderivat IV, 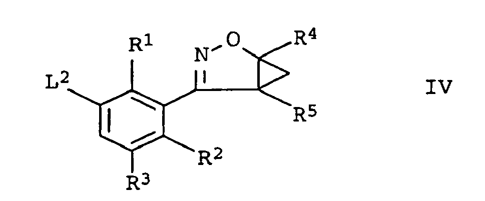 wobei die Variablen R¹ bis R⁵ die in Anspruch 1 angegebene Bedeutung haben und L² für eine Abgangsgruppe steht, in Gegenwart von Kohlenmonoxid, eines Katalysators sowie einer Base umsetzt und das Kondensationsprodukt gegebenenfalls mit einer Verbindung der Formel X
L⁴-R^{6a} X
umsetzt, worin L⁴ und R^{6a} die in Anspruch 4 angegebene Bedeutung haben.

6. Verfahren zur Herstellung von Cyclopropyl-anellierten 3-(4,5-Dihydroisoxazol-3-yl)-substituierten Benzoylpyrazolen nach Anspruch 1, bei dem man ein 3-(5-Halogenmethyl-4,5-dihydroisoxazol-3-yl)-substituiertes Benzoylpyrazol der Formel XI 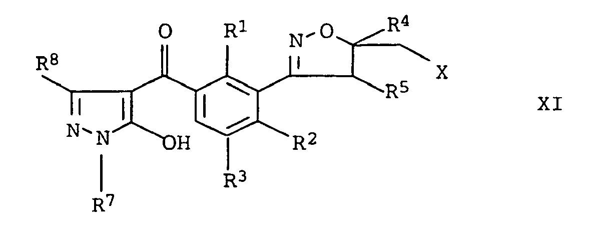 worin R¹ bis R⁵, R⁷ und R⁸ die in Anspruch 1 angegebene Bedeutung haben und X für Halogen steht, mit einer Base zu einer Verbindung der Formel I, worin R⁶ für Hydroxy steht, umsetzt und das Umsetzungsprodukt gegebenenfalls mit einer Verbindung der Formel X
L⁴-R^{6a} X
umsetzt, worin L⁴ und R^{6a} die in Anspruch 4 angegebene Bedeutung haben.

7. Verbindung der Formel IIIa oder IV 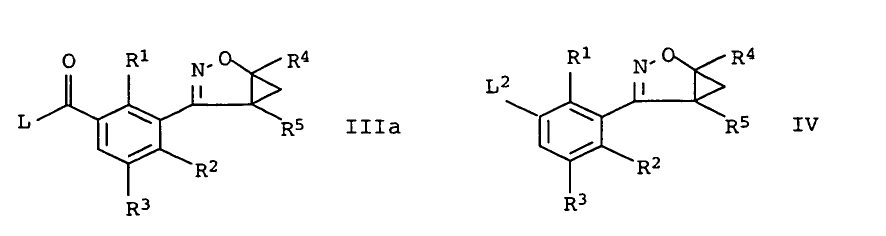 worin R¹ bis R⁵ die in Anspruch 1 angegebene Bedeutung haben, L für Hydroxy oder einen abhydrolysierbaren Rest und L² für eine nucleophil verdrängbare Abgangsgruppe steht.

8. Mittel, enthaltend eine herbizid wirksame Menge mindestens eines Cyclopropyl-anellierten 3-(4,5-Dihydroisoxazol-3-yl)-substituierten Benzoylpyrazols der Formel I oder eines landwirtschaftlich brauchbaren Salzes davon gemäß einem der Ansprüche 1 bis 3 und für die Formulierung von Pflanzenschutzmitteln übliche Hilfsmittel.

9. Verfahren zur Bekämpfung von unerwünschtem Pflanzenwuchs, bei dem man eine herbizid wirksame Menge mindestens eines Cyclopropyl-anellierten 3-(4,5-Dihydroisoxazol-3-yl)-substituierten Benzoylpyrazols der Formel I oder eines landwirtschaftlich brauchbaren Salzes davon gemäß einem der Ansprüche 1 bis 3 auf Pflanzen, deren Lebensraum und/oder auf Samen einwirken lässt.

10. Verwendung von Cyclopropyl-anellierten 3-(4,5-Dihydroisoxazol-3-yl)-substituierten Benzoylpyrazolen der Formel I und/oder deren landwirtschaftlich brauchbaren Salzen gemäß einem der Ansprüche 1 bis 3 als Herbizide.

## Claims

1. A cyclopropyl-fused 3-(4,5-dihydroisoxazol-3-yl)-substituted benzoylpyrazole of the formula I 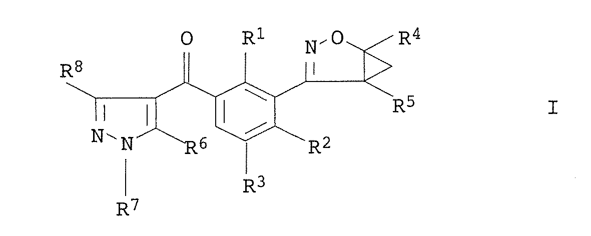 in which
R¹ is C₁-C₆-alkyl, C₁-C₆-haloalkyl, C₁-C₆-alkoxy, C₁-C₆-haloalkoxy, halogen or nitro;
R² is C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₁-C₆-alkylsulfinyl, C₁-C₆-haloalkylsulfinyl, C₁-C₆-alkylsulfonyl, C₁-C₆-haloalkylsulfonyl, halogen, cyano or nitro;
R³ is hydrogen, C₁-C₆-alkyl or halogen;
R⁴ is hydrogen, C₁-C₄-alkyl or C₁-C₄-haloalkyl ;
R⁵ may have the meanings given for R⁴ ; or
R⁴, R⁵ together are a C₁-C₄-alkanediyl group which may be partially or fully halogenated and/or may carry one to three C₁-C₄-alkyl groups;
R⁶ is hydroxyl, C₁-C₆-alkoxy, C₃-C₆-alkenyloxy, C₁-C₆-alkylsulfonyloxy, C₁-C₆-alkylcarbonyloxy, phenyl-C₁-C₄-alkoxy, phenylcarbonyl-C₁-C₄-alkoxy, phenylsulfonyloxy, phenylcarbonyloxy, where the phenyl radical of the four lastmentioned substituents may be partially or fully halogenated and/or may carry one to three of the following groups: nitro, cyano, C₁-C₄-alkyl, C₁-C₄-haloalkyl, C₁-C₄-alkoxy or C₁-C₄-haloalkoxy;
R⁷ is hydrogen, C₁-C₆-alkyl or cyclopropyl;
R⁸ is hydrogen, C₁-C₆-alkyl or C₁-C₆-haloalkyl;
and its agriculturally useful salts.

2. The cyclopropyl-fused 3-(4,5-dihydroisoxazol-3-yl)-substituted benzoylpyrazole according to claim 1 in which
R¹ is C₁-C₆-alkyl or halogen;
R² is C₁-C₆-haloalkyl, C₁-C₆-alkylsulfonyl or halogen;
R³ is hydrogen, C₁-C₄-alkyl or halogen;
R⁷ is C₁-C₄-alkyl or cyclopropyl;
R⁸ is hydrogen or C₁-C₄-alkyl.

3. The cyclopropyl-fused 3-(4,5-dihydroisoxazol-3-yl)-substituted benzoylpyrazole according to claim 1 or 2 in which R⁶ is hydroxyl, phenyl-C₁-C₂-alkoxy, phenylcarbonyl-C₁-C₂-alkoxy, phenylsulfonyloxy or phenylcarbonyloxy, where the phenyl radical of the four lastmentioned substituents may be partially or fully halogenated and/or may carry one to three of the following groups: nitro, cyano, C₁-C₄-alkyl, C₁-C₄-haloalkyl, C₁-C₄-alkoxy or C₁-C₄-haloalkoxy.

4. A process for preparing cyclopropyl-fused 3-(4,5-dihydroisoxazol-3-yl)-substituted benzoylpyrazoles according to claim 1, which comprises acylating a pyrazole of the formula II 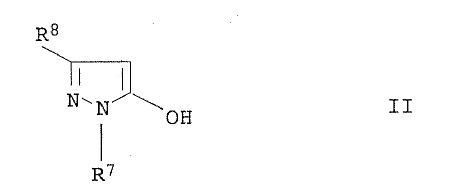 with a benzoic acid derivative of the formula III, 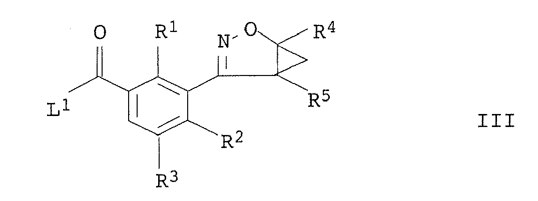 in which the variables R¹ to R⁵, R⁷ and R⁸ are as defined in claim 1 and L¹ is hydroxyl or a nucleophilically displaceable leaving group;
rearranging the acylation product to form a compound of the formula I in which R⁶ is hydroxyl and, if appropriate, reacting the product of the rearrangement with a compound of the formula X
L⁴-R^{6a} X
in which
L⁴ is a nucleophilically displaceable leaving group; and
R^{6a} is C₁-C₆-alkyl, C₃-C₆-alkenyl, C₁-C₆-alkylsulfonyl, C₁-C₆-alkylcarbonyl, phenyl-C₁-C₄-alkyl, phenylcarbonyl-C₁-C₄-alkyl, phenylsulfonyl or phenylcarbonyl, where the phenyl radical of the four lastmentioned substituents may be partially or fully halogenated and/or may carry one to three of the following groups: nitro, cyano, C₁-C₄-alkyl, C₁-C₄-haloalkyl, C₁-C₄-alkoxy or C₁-C₄-haloalkoxy.

5. A process for preparing cyclopropyl-fused 3-(4,5-dihydroisoxazol-3-yl)-substituted benzoylpyrazoles according to claim 1, which comprises reacting a pyrazone of the formula II 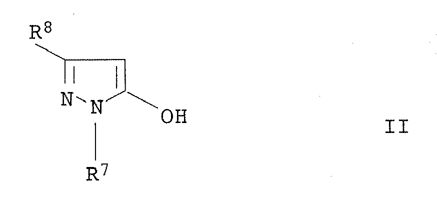 in which the variables R⁷ and R⁸ are as defined in claim 1, or an alkali metal salt thereof, with a cyclopropyl-fused 3-(4,5-dihydroisoxazol-3-yl)-benzene derivative IV 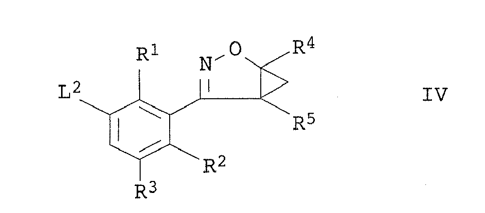 where the variables R¹ to R⁵ are as defined in claim 1 and L² is a leaving group in the presence of carbon monoxide, a catalyst and a base and, if appropriate, reacting the condensation product with a compound of the formula X
L⁴-R^{6a} X
in which L⁴ and R^{6a} are as defined in claim 4.

6. A process for preparing cyclopropyl-fused 3-(4,5-dihydroisoxazol-3-yl)-substituted benzoylpyrazoles according to claim 1, which comprises reacting a 3-(5-halomethyl-4,5-dihydroisoxazol-3-yl)-substituted benzoylpyrazole of the formula XI 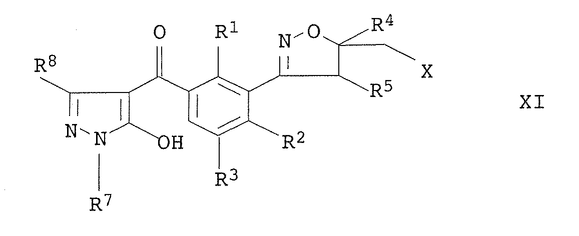 in which R¹ to R⁵, R⁷ and R⁸ are as defined in claim 1 and X is halogen with a base to give a compound of the formula I in which _{R}⁶ is hydroxyl and, if appropriate, reacting the reaction product with a compound of the formula X
L⁴-R^{6a} X
in which L⁴ and R^{6a} are as defined in claim 4.

7. A compound of the formula IIIa or IV 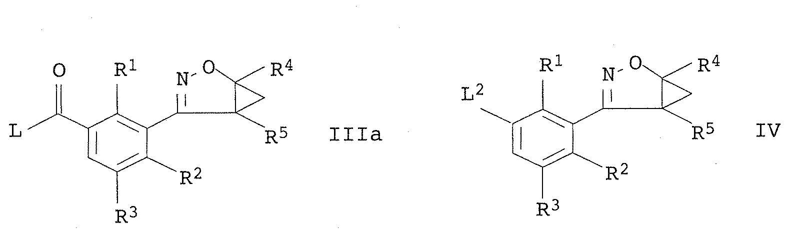 in which R¹ to R⁵ are as defined in claim 1, L is hydroxyl or a radical which can be removed by hydrolysis and L² is a nucleophilically displaceable leaving group.

8. A composition, comprising a herbicidally effective amount of at least one cyclopropyl-fused 3-(4,5-dihydroisoxazol-3-yl)-substituted benzoylpyrazole of the formula I or an agriculturally useful salt thereof according to any of claims 1 to 3 and auxiliaries customarily used for formulating crop protection agents.

9. A method for controlling undesirable vegetation, which comprises allowing a herbicidally effective amount of at least one cyclopropyl-fused 3-(4,5-dihydroisoxazol-3-yl)-substituted benzoylpyrazole of the formula I or an agriculturally useful salt thereof according to any of claims 1 to 3 to act on plants, their habitat and/or on seeds.

10. The use of cyclopropyl-fused 3-(4,5-dihydroisoxazol-3-yl)-substituted benzoylpyrazoles of the formula I and/or agriculturally useful salts thereof according to any of claims 1 to 3 as herbicides.

## Revendications

1. Benzoylepyrazole substitué par du 3-(4,5-dihydroisoxazol-3-yle)- cyclisé au cyclopropyle selon la formule I 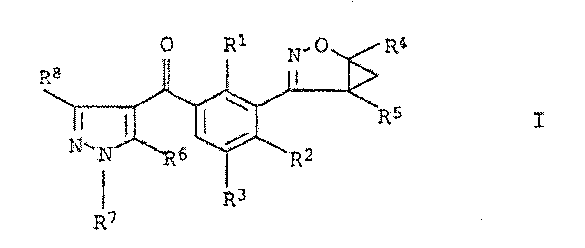 dans laquelle :
R¹ représente un alkyle en C₁-C₆, un alkyle halogéné en C₁-C₆, un alcoxy en C₁-C₆, un alcoxy halogéné en C₁-C₆, un groupe halogène ou nitro ;
R² représente un alkyle en C₁-C₆, un alkyle halogéné en C₁-C₆, un alcoxy en C₁-C₆, un alcoxy halogéné en C₁-C₆, un thioalkyle halogéné en C₁-C₆, un thio-halogénure d'alkyle en C₁-C₆, un sulfinyle d'alkyle en C₁-C₆, un sulfinyle d'alkyle halogéné en C₁-C₆, un sulfonyle d'alkyle en C₁-C₆, un sulfonyle d'alkyle halogéné en C₁-C₆, un groupe halogène, cyano ou nitro ;
R³ représente de l'hydrogène, un alkyle en C₁-C₆ ou un groupe halogène ;
R⁴ représente de l'hydrogène, un alkyle en C₁-C₄ ou un halogénure d'alkyle en C₁-C₄ ;
R⁵ peut prendre les significations données pour R⁴ ; ou
R⁴, R⁵ représentent ensemble un groupe alcandiyle en C₁-C₄, qui peut être partiellement ou complètement halogéné et/ou peut porter un à trois groupes alkyle en C₁-C₄ ;
R⁶ représente un groupe hydroxy, un alcoxy en C₁-C₆, un alcényloxy en C₃-C₆, un alkyle-sulfonyloxy en C₁-C₆, un alkyle-carbonyloxy en C₁-C₆, un phényle-alcoxy en C₁-C₄, un phénylcarbonyle-alcoxy en C₁-C₄, un phénylsulfonyloxy, un phénylcarbonyloxy, le radical phényle des quatre substituants dénommés en dernier lieu pouvant être halogéné partiellement ou complètement et/ou pouvant porter un à trois des groupes suivants : nitro, cyano, alkyle en C₁-C₄, halogénure d'alkyle en C₁-C₄, alcoxy en C₁-C₄ ou alcoxy halogéné en C₁-C₄ ;
R⁷ représente de l'hydrogène, un alkyle en C₁-C₆ ou un cyclopropyle ;
R⁸ représente de l'hydrogène, un alkyle en C₁-C₆ ou un halogénure d'alkyle en C₁-C₆ ;
de même que leurs sels utilisables dans l'agriculture.

2. Benzoylepyrazole substitué par du 3-(4,5-dihydroisoxazol-3-yle)- cyclisé au cyclopropyle selon la revendication 1, dans lesquels:
R¹ représente un alkyle en C₁-C₆ ou un groupe halogène ;
R² représente un halogénure d'alkyle en C₁-C₆, un alkylsulfonyle en C₁-C₆ ou un groupe halogène ;
R³ représente de l'hydrogène, un alkyle en C₁-C₄ ou un halogène ;
R⁷ représente un alkyle en C₁-C₄ ou un cyclopropyle ;
R⁸ représente de l'hydrogène ou un alkyle en C₁-C₄.

3. Benzoylepyrazole substitué par du 3-(4,5-dihydroisoxazol-3-yle)- cyclisé au cyclopropyle selon la revendication 1 ou 2, dans lesquels R⁶ représente un groupe hydroxy, un phényle-alcoxy en C₁-C₂, un phénylcarbonyle-alcoxy en C₁-C₂, un phénylsulfonyloxy ou un phénylcarbonyloxy, le radical phényle des quatre substituants dénommés en dernier lieu pouvant être halogéné partiellement ou complètement et/ou pouvant porter un à trois des groupes suivants : nitro, cyano, alkyle en C₁-C₄, halogénure d'alkyle en C₁-C₄, alcoxy en C₁-C₄ ou alcoxy halogéné en C₁-C₄.

4. Benzoylepyrazole substitué par du 3-(4,5-dihydroisoxazol-3-yle)- cyclisé au cyclopropyle selon la revendication 1, dans lequel on acyle un pyrazol selon la formule II 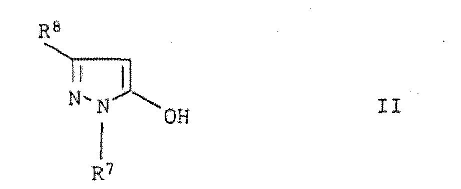 avec un dérivé de l'acide benzoïque selon la formule III, 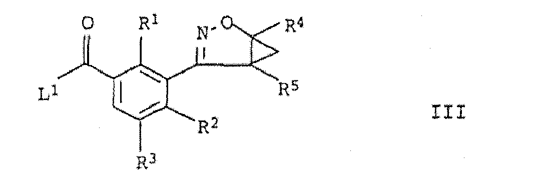 dans laquelle les variables R¹ à R⁵, R⁷ et R⁸ ont la signification indiquée dans la revendication 1 et L¹ représente un groupe hydroxy ou un groupe partant à substitution nucléophile ;
on transpose le produit d'acylation dans un composé selon la formule I, dans laquelle R⁶ représente un groupe hydroxy, et on fait réagir le produit de transposition, éventuellement avec un composé selon la formule X
L⁴-R^{6a} X
dans laquelle :
L⁴ représente un groupe de départ à substitution nucléophile ; et
R^{6a} représente un alkyle en C₁-C₆, un alcényle en C₃-C₆, un alkylsulfonyle en C₁-C₆, un alkylcarbonyle en C₁-C₆, un phénylalkyle en C₁-C₄, un phénylcarbonyle-alkyle en C₁-C₄, un phénylsulfonyle ou un phénylcarbonyle, le radical phényle des quatre substituants mentionnés en dernier lieu pouvant être partiellement ou complètement halogéné et/ou pouvant porter un à trois des groupes suivants : nitro, cyano, alkyle en C₁-C₄, halogénure d'alkyle en C₁-C₄, alcoxy en C₁-C₄ ou alcoxy halogéné en C₁-C₄.

5. Procédé pour la préparation de benzoylepyrazole substitué par du 3-(4,5-dihydroisoxazol-3-yle)-cyclisé au cyclopropyle selon la revendication 1, dans lequel on fait réagir un pyrazol selon la formule II 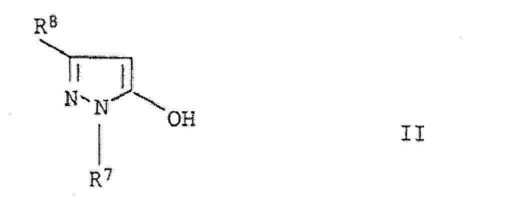 dans laquelle les variables R⁷ et R⁸ ont la signification mentionnée dans la revendication 1, ou un de ses sels alcalins, avec un dérivé IV de 3-(4,5-dihydroisoxazol-3-yle-benzène IV cyclisé au cyclopropyle, 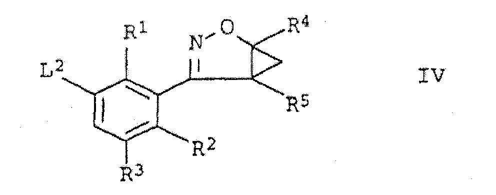 dans laquelle les variables R¹ à R⁵ ont la signification indiquée dans la revendication 1, et L² représente un groupe partant, en présence de monoxyde de carbone, d'un catalyseur ainsi que d'une base et l'on fait réagir le produit de condensation éventuellement avec un composé selon la formule X
L⁴-R^{6a} X
dans laquelle L⁴ et R^{6a} ont la signification indiquée dans la revendication 4.

6. Procédé pour la préparation de benzoylepyrazole substitué par du 3-(4,5-dihydroisoxazol-3-yle)-cyclisé au cyclopropyle selon la revendication 1, dans lequel on fait réagir un benzoylepyrazole substitué par du 3-(5- halogéno-méthyle-4,5-dihydroxyisoxazol-3-yle)-cyclisé selon la formule XI 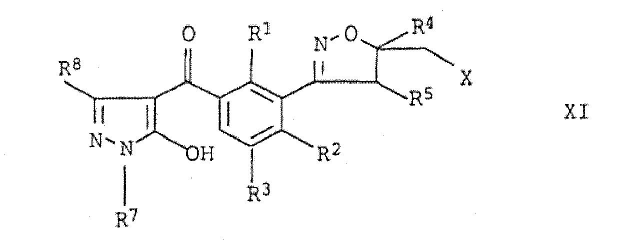 dans laquelle R¹ à R⁵, R⁷ et R⁸ ont la signification indiquée dans la revendication 1 et X représente un groupe halogène, avec une base pour donner un composé selon la formule I, dans laquelle R⁶ représente un groupe hydroxy, et l'on fait réagir le produit réactionnel éventuellement avec un composé selon la formule X
L⁴-R^{6a} X
dans laquelle L⁴ et R^{6a} ont la signification indiquée dans la revendication 4.

7. Composé selon la formule IIIa ou IV 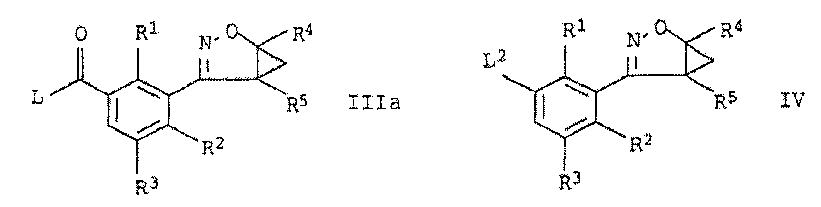 dans laquelle R¹ à R⁵ ont la signification indiquée dans la revendication 1, L représente un groupe hydroxy ou un radical hydrolysable et L² représente un groupe de départ à substitution nucléophile.

8. Agent comprenant une quantité herbicidement efficace d'au moins un benzoylepyrazole substitué par du 3-(4,5-dihydroisoxazol-3-yle)- cyclisé au cyclopropyle selon la formule I ou d'un sel utilisable en agriculture de celui-ci, conformément à l'une des revendications 1 à 3 pour la formulation d'auxiliaires usuels de produits phytosanitaires.

9. Procédé pour la lutte contre la croissance des végétaux indésirables, dans lequel on fait agir une quantité herbicidement efficace d'au moins un benzoylepyrazole substitué par du 3-(4,5-dihydroisoxazol-3-yle)- cyclisé au cyclopropyle selon la formule I ou d'un sel utilisable en agriculture selon l'une des revendications 1 à 3 sur des végétaux dont l'espace vital et/ou les graines peuvent être influencés.

10. Utilisation comme herbicides de benzoylepyrazole substitué par du 3-(4,5-dihydroisoxazol-3-yle)-cyclisé au cyclopropyle selon la formule I et/ou leurs sels utilisables en agriculture suivant l'une des revendications 1 à 3.
